# EUROPEAN PATENT APPLICATION

(11) **EP 1 221 481 A1**
(43) Date of publication of application: **10.07.2002**
(21) Application number: 00966474.9
(22) Date of filing: 13.10.2000
(51) Int. Cl.: C12N 15/10, C12P 21/00, C12M 1/00

(54) **TEMPLATE MOLECULE HAVING BROAD APPLICABILITY AND HIGHLY EFFICIENT FUNCTION MEANS OF CELL-FREE SYNTHESIS OF PROTEINS BY USING THE SAME**

(30) Priority: 15.10.1999 JP 29437099; 18.07.2000 WO PCT/JP00/04814
(71) Applicant: Wakenyaku Co., Ltd., Kyoto-shi, Kyoto 606-8171 (JP); Endo, Yaeta, Matsuyama-shi, Ehime 791-8016 (JP)
(72) Inventor: ENDO, Yaeta, Matsuyama-shi, Ehime 791-8016 (JP)
(74) Representative: Forstmeyer, Dietmar, Dr. rer. nat., Dipl.-Chem.
(86) International application number: JP0007123
(87) International publication number: WO0127260

(57) **Abstract**

A sequence having a specific sequence and a sequence structure having at least an non-translational sequence are introduced into 5'-terminal and 3'-terminal, respectively, of mRNA which is a protein translational template molecule whereby mRNA having a high translation efficiency is constructed; a plasmid having a broad applicability containing a template DNA for transcription of the said mRNA is constructed; a synthetic method and a purifying method for the said mRNA having a high translational template activity are constructed; a continuous transcriptional/translational non-coupling cell-free protein synthesis where a continuous RNA synthetic means and a continuous cell-free protein synthetic means utilizing a wheat germ extract are combined is constructed as a practical cell-free protein synthetic means using the same; and a method for a co-expression of plural proteins utilizing a cell-free protein synthetic means is constructed.

SEQUENCE LISTING

<110> ENDO, Yaeta
      WAKENYAKU CO., LTD

<120> Template molecule having broad applicability and highly
      efficient function and cell-free protein synthetic
      means using the same

<130> GP00-1006-2

<140> PCT/JP00/07123
<141> 2000-10-13

<150> JP P H11-294370
<151> 1999-10-15

<160> 3

<170> PatentIn Ver. 2.1

<210> 1
<211> 34
<212> RNA
<213> Artificial Sequence

<220>
<223> Description of Artificial Sequence:Designed
      polynucleotide used as a 5' untranslated region

<400> 1
gaacaauuac cauacauuuu acauuacaac uaca                                34

<210> 2
<211> 29

<212> RNA
<213> Artificial Sequence

<220>
<223> Description of Artificial Sequence:Designed
      polynucleotide used as a 5' untranslated region

<400> 2
gaacaacaac aacaacaaac aacaacaaa                                    29


<210> 3
<211> 15
<212> RNA
<213> Artificial Sequence

<220>
<223> Description of Artificial Sequence:Designed
      polynucleotide used as a 5' untranslated region

<400> 3
ggacaacaac aacaa                                                   15

## Description

### Technical Field

The present invention is to provide design of translational template molecule having a high efficiency and a broad applicability, construction of plasmid having a broad applicability for the synthesis of RNA, means for the synthesis of RNA, means for supplying of RNA to a cell-free protein synthetic system and means for the transcriptional/translational non-coupling continuous cell-free protein synthesis where those techniques are combined with an object of developing a cell-free protein synthetic system using an extract prepared from cells and tissues to a practical art.

### Background Art

In the fields such as development of pharmaceuticals and plant breeding in agriculture, it is an important matter that functions of many proteins *in vivo* are made clear. Especially shortly before the completion of genomic plan, the matter to be solved has now been quickly developing from analysis of structure of gene to analysis of function of gene. As one of the methods for analysis of function of gene, there is a method where protein for which gene is coding is produced and the function of the said protein is analyzed.

Synthesis of protein by cell consists of the steps comprising that genetic information stored in DNA is firstly transcribed to mRNA which is a translational template molecule and then amino acids are bound by a translation device comprising a group of factors such as ribosome according to the information described in the base sequence of this mRNA whereupon protein which is a translation product of the genetic information is synthesized.

With regard to a method where a protein synthesis reaction which efficiently proceeds in cells is carried out *in vitro* for the production of protein for which gene is coding, there have been vigorously carried out the studies for a cell-free protein synthetic method where, for example, ribosome which is a protein translation device inherent in cells and the like are extracted from living organism and to this extract are added translational template, amino acids which are to be substrates, energy source, various ions, buffer and other effective factors whereupon protein is synthesized *in vitro* (Japanese Patent Laid-Open Nos. 06/98790, 06/225783, 07/194, 09/291 and 07/147992). *Escherichia coli,* wheat germ, reticulocytes of rabbit, etc. have been used for the preparation of extract of cells or living body tissues for the protein synthesis used for the reaction system of a cell-free protein synthesis or, in other words, a cell-free protein synthetic system.

The cell-free protein synthetic system retains a property which is as high as living cells in terms of peptide synthetic rate and precision of translation reaction and, therefore, there is no need of complicated chemical reaction steps or troublesome cell incubation steps whereby development of its practical system has been carried out. Usually however, protein synthetic ability of the cell extract which is extracted from cells of living organisms is quite unstable and, therefore, its protein synthetic efficiency is low and, further, lowering of quality of the cell extract during preservation is significant whereby the quantity of the synthesized product obtained by the cell-free protein synthetic system is in such a low level as the degree which is detectable by means of radioisotope labeling and that is unable to be utilized as a practical production means for proteins.

The present inventor has previously proposed methods for solving the disadvantage in the conventional cell-free protein synthetic system or, in other words, a cell extract preparation for a cell-free protein synthesis and a cell-free protein synthetic means (PCT/JP99/04088).

However, in carrying out the synthesis of proteins efficiently, it is important in addition to the above-mentioned invention that the translation efficiency of mRNA which is a translational template is enhanced. In the conventional synthesis of mRNA having a high translation efficiency, it is necessary that a special structure which is called a CAP structure such as 7mGpppG is linked to its 5' -terminal. However, since this CAP molecule *per se* have a strong inhibiting activity to the initiation reaction for the protein synthesis, it is essential that, after the synthesis of mRNA, the residual molecules are completely removed from the mRNA fractions. In addition, when the amount of mRNA having a CAP structure at the 5'-terminal is made in a high concentration, the protein synthetic reaction is inhibited and, therefore, the concentration is to be made low whereby the efficiency of synthesis of proteins is low as well. Further, CAP molecule is expensive and, for a practical utilization of a cell-free protein synthetic means, those are left as the problems to be solved.

Generally, an *in vitro* RNA synthesis is carried out in a closed reaction system (closed system) such as in a test tube in the presence of four types of ribonucleoside-5' -triphosphate as substrates, RNA polymerase, spermidine and magnesium ion as well as an appropriate buffer. Further, it is necessary for isolation and purification of RNA such as mRNA, protein is removed from the synthetic reaction solution by means of a phenolic treatment and then residual ribonucleoside and pyrophosphoric acid which is a by-product of the synthetic reaction (and also CAP molecule having a stronger translation inhibiting action) are removed from the RNA fractions. Needless to say, such synthetic and purifying processes should be carried out in the absence of RNase and the said processes require sufficient experience for the operation and are troublesome as well. Furthermore, in the RNA synthesis reaction in the conventional closed system, substrate concentrations lower as the synthetic reaction proceeds and, at the same time, by-produced pyrophosphoric acid is accumulated whereby the RNA synthesis reaction reaches chemical equilibrium relatively quickly and stops and, accordingly, the synthetic yield of RNA is principally low. In such a synthetic method, the reaction stops within about two hours for example and amount of the resulting RNA is about 300 µg per ml of the reaction volume which is not efficient.

On the other hand, in order to make the protein synthesis efficient, a transcriptional/translational coupled cell-free protein synthetic system where mRNA synthesis by transcription and protein synthesis by translation are coupled so that transcription and translation are simultaneously carried out in the same system was firstly reported in a cell-free protein synthetic system using a cell extract (S-30) of *Escherichia coli* which is a prokaryote (Zubay, G., (1973), *Ann. Rev. Genet.,* 7, 267-287), then that was also reported in a cell-free protein system using a cell extract of eukaryote (Roberts, B. et al., (1975), *Proc. Natl. Acad. Sci. USA,* 72, 1922-1926; Pelham, H. R. B. et al., (1978), *Eur. J. Biochem.,* 82, 199-209; and U. S. Patents No. 5,665,563, No. 5,492,817 and No. 5,324,637) and they have been available in the market as well. However, in the transcription reaction and the translation reaction, there are big differences in optimum ion concentrations and optimum concentrations of ribonucleoside-5'-triphosphate which is a substrate for the RNA synthesis, particularly, adenosine-5'-triphosphate (ATP) and guanosine-5'-triphosphate (GTP) which are energy for the translation whereby the protein synthesis efficiency in the conventional transcriptional/translational coupling system is quite low. Furthermore, in such a system using the cells of an eukaryote, CAP molecule *per se* has a strong translation inhibiting action and, therefore, it is principally impossible to construct a highly efficient translation system coupling with the synthesis of mRNA having a CAP structure at 5'-terminal.

In addition, proteins do not function solely in cells but achieve the functions as a result of interaction with various kinds of proteins, nucleic acids, low-molecular species and/or cell membrane components. It is also an important thing in the field of development of pharmaceuticals that the said interaction is made clear. With regard to a method for investigating the function of gene or gene group, there have been known a gene disruption method of incubated cells and living individuals and a two-hybrid method (Field, S., (1993), *METHODS: A companion to Meth. Enzymol.*, 5, 116-124). However, the gene disruption method of incubated cells and living individuals has a limitation that, as a result of observation of growth of cells or individuals, the reactions occurring in the cells can be investigated only indirectly. Further, a two-hybrid method is able to co-express plural proteins simultaneously and is utilized for the analysis of interaction among proteins but that is a just investigational experimental means whereby the operation is troublesome and time-consuming and, further, the obtained result is indirect as well. On the other hand, in the conventional genetic engineering means using living cells, it is possible to co-express about two kinds of proteins but no technique for an efficient co-expression of many proteins has been developed yet.

### Disclosure of the Invention

The object to be solved by the present invention is to design a translational template molecular mRNA having high efficiency and broad applicability and is to provide construction of plasmid having broad applicability for the synthesis of mRNA which can be easily prepared and synthetic and purifying means for mRNA.

One of other objects of the present invention is to provide an efficiently supplying means of mRNA to a cell-free protein synthetic system.

Another object of the present invention is to provide a continuous and highly efficient transcriptional/translational non-coupling cell-free protein synthetic means in which the above-mentioned elements and techniques are combined.

Still another object of the present invention is to provide a method for the co-expression of plural proteins in a cell-free protein synthetic system.

The present inventor has carried out an investigation for developing a practical cell-free protein synthetic means having a broad applicability and high efficiency and, as a result, a base sequence having an Ω sequence of tobacco mosaic virus (TMV), a base sequence having a leader sequence of alfalfa virus (AMV) or three kinds of short base sequences derived therefrom (SEQ ID NO: 1, SEQ ID NO: 2 and SEQ ID NO: 3 of the Sequence Listing) is introduced into 5'-terminal of mRNA which is a protein translational template molecule and a sequence structure having at least non-translational sequence into 3'-terminal thereof whereupon mRNA having a high translation efficiency is constructed, a plasmid having a broad applicability containing the transcriptional template DNA of the said constructed mRNA is constructed and there are provided a method for the synthesis and purification of the said constructed mRNA having a high translational template activity and a continuous transcriptional/translational non-coupling system with a continuous cell-free protein synthesis utilizing mRNA synthesis and wheat germ extract as a practical cell-free protein synthetic means using the same whereupon the present invention has been achieved.

Thus, the present invention is as follows.
1. A modifying means for translational template molecule which is a modifying means for translational template molecule being able to be used as a means for cell-free protein synthesis comprising that at least one base sequence selected from the following, i.e.
   1) a base sequence having an alfalfa mosaic virus (AMV) leader sequence,
   2) a base sequence having an Ω sequence of tobacco mosaic virus (TMV),
   3) a base sequence having 70% or more homology to an alfalfa mosaic virus (AMV) leader sequence and being able to give mRNA which has about 70% or more translation initiation activity as compared with the translation initiation activity of mRNA where a CAP structure is present,
   4) a base sequence having 70% or more homology to an Ω sequence of tobacco mosaic virus (TMV) and being able to give mRNA which has about 70% or more translation initiation activity as compared with the translation initiation activity of mRNA where a CAP structure is present,
   5) a base sequence described in SEQ ID NO: 1, SEQ ID NO: 2 or SEQ ID NO: 3 of the Sequence Listing and
   6) a base sequence having 70% or more homology to the sequence selected from the base sequences described in SEQ ID NO: 1 to NO: 3 of the Sequence Listing and being able to give mRNA which has about 70% or more translation initiation activity as compared with the translation initiation activity of mRNA where a CAP structure is present
      is introduced into a structure of 5'-terminal of the said translational template molecule and then at least a non-translational sequence is introduced into a structure of 3'-terminal.
2. The modifying means for translational template molecule according to the above 1, wherein there is combined with an introduction of a base sequence having a known translation initiation activity into a 5'-terminal.
3. A plasmid utilized for the manufacture of a translational template molecule used for a cell-free protein synthetic means wherein
   ① a base sequence carrying a promoter function,
   ② a base sequence at the downstream thereof which is to be a transcriptional template for at least
      an alfalfa mosaic virus (AMV) leader sequence,
      an Ω sequence of tobacco mosaic virus (TMV),
      a base sequence having 70% or more homology to an alfalfa mosaic virus (AMV) leader sequence or an Ω sequence of tobacco mosaic virus (TMV) and being able to give mRNA having about 70% or more translation initiation activity as compared with the translation initiation activity of mRNA where a CAP structure is present,
      a base sequence described in SEQ ID NO: 1, SEQ ID NO: 2 or SEQ ID NO: 3 of the Sequence Listing or
      a base sequence having 70% or more homology to the base sequence described in SEQ ID NO: 1, SEQ ID NO: 2 or SEQ ID NO: 3 of the Sequence Listing and being able to give mRNA which has about 70% or more translation initiation activity as compared with the translation initiation activity of mRNA where a CAP structure is present,
   ③ plural restriction enzyme cleavage sites at the region corresponding to the downstream thereof and
   ④ at least 3'-terminal non-translational sequence at the region corresponding to the downstream of the termination codon of ORF (open reading frame)
      are aligned.
4. The plasmid according to the above 3, wherein the size of the plasmid body is 3 Kb or more,
5. The plasmid according to the above 3 or 4, wherein the plasmid is a circular type,
6. The plasmid according to the above 5, wherein a base sequence carrying a transcription terminator function is aligned at the upstream of a base sequence carrying a promoter function.
7. The plasmid according to the above 3 or 4, wherein the plasmid is a straight-chain type .
8. A method for the synthesis of RNA utilizing the modifying means according to the above 1 or 2.
9. The method for the synthesis of RNA according to the above 8, wherein it is characterized in that, in a reaction system for the synthesis of RNA, a substance necessary for the synthesis of the said RNA is continuously supplied and/or a substance inhibiting the synthesis of the said RNA and the by-product of the reaction are continuously removed.
10. A method for the synthesis of RNA used for a cell-free protein synthetic means using any of the plasmids according to the above 3 to 7.
11. The method for the synthesis of RNA according to the above 10, wherein it is characterized in that, in a reaction system for the synthesis of RNA, a substance necessary for the synthesis of the said RNA is continuously supplied and/or a substance inhibiting the synthesis of the said RNA is continuously removed.
12. A cell-free protein synthetic means utilizing the mRNA obtained by any of the synthetic methods for RNA according to the above 8 to 11.
13. A cell-free protein synthetic means utilizing any of the plasmids according to the above 3 to 7.
14. A cell-free protein synthetic means where the cell-free protein synthetic means according to the above 12 or 13 is a cell-free protein synthetic means using an extract of wheat germ and, in the said wheat germ extract, contamination of endosperm components is substantially removed.
15. A continuous transcriptional/translational non-coupling cell-free protein synthetic means which is the cell-free protein synthetic means according to the above 12 or 13, wherein it is characterized in that the mRNA synthesis (transcription) and the protein synthesis (translation) are not coupled but are continuously carried out in a system.
16. A continuous transcriptional/translational non-coupling cell-free protein synthetic means where the protein synthesis (translation) can be carried out in the same reaction vessel which is a cell-free protein synthetic means utilizing the plasmid according to the above 5 or 6 comprising that the mRNA synthesis (transcription) is carried out in a reaction vessel having a molecular sieve carrier, the said synthesized mRNA is purified and collected by a carrier which can carry out a molecular sieving and, under a dialysis condition, temperature of the reaction solution containing mRNA in the reaction vessel is changed to a temperature appropriate for the protein synthesis so that the protein synthetic reaction of the mRNA is carried out.
17. A continuous transcriptional/translational non-coupling cell-free protein synthetic means which is a cell-free protein synthetic means utilizing the plasmid according to the above 5 or 6, wherein, a reaction vessel having a dialyzing means is used and, after the mRNA synthesis (transcription) is carried out in an inner area of dialysis membrane, the temperature of the reaction solution is changed to a temperature which is appropriate for the protein synthesis (translation) and, the solution composition in the inner area of dialysis membrane is converted to a solution composition for the protein synthesis by dialysis whereby the mRNA synthesis (transcription) and the protein synthesis (translation) can be carried out in the same reaction vessel.
18. A continuous transcriptional/translational non-coupling cell-free protein synthetic means comprising the following steps:
   (a) mRNA is synthesized in a reaction vessel having a carrier which is able to carry out a molecular sieving,
   (b) the said synthesized mRNA is purified by the carrier which is able to carry out a molecular sieving and then collected and
   (c) the protein synthesis is carried out in a reaction vessel containing the said synthesized and collected mRNA under a dialysis condition.
19. A continuous transcriptional/translational non-coupling cell-free protein synthetic means comprising the following steps:
   (1) there is used a reaction vessel having a dialysis membrane where a mixed solution of the transcription reaction solution and the translation reaction solution is added to the inner area of the dialysis membrane,
   (2) a solution for the protein synthesis reaction is filled in as an external solution for dialysis to manufacture the mRNA in a permeating membrane,
   (3) then the temperature of the reaction solution is changed to a temperature which is suitable for the protein synthesis reaction (translation reaction) and
   (4) synthesis of protein is carried out under a dialysis condition.
20. A continuous transcriptional/translational non-coupling cell-free protein synthetic means where, in the continuous transcriptional/translational non-coupling cell-free protein synthetic means in any of the above 15 to 19, an element which is selected at least from mRNA which is to be a translational template for protein synthesis reaction, enzyme of an energy regeneration system, substrate and energy source is subjected to a treatment selected from addition, preservation, exchange and discharge.
21. A cell-free protein synthetic means, characterized in that, a protein coating is applied at least on the surface participating in the dialyzing means or the molecular sieving means among the surfaces which are contacting to the reaction solution in the reaction vessel wherein mRNA synthesis is carried out.
22. The continuous transcriptional/translational non-coupling cell-free protein synthetic means according to any of the above 16, 18, 20 and 21, wherein, as a molecular sieving means or a carrier which is able to carry out a molecular sieving, there is carried out using a filter having a pore size whereby separation of the synthesized mRNA from low-molecular substances such as substrate and by-products is possible.
23. A continuous transcriptional/translational non-coupling cell-free protein synthetic means wherein the cell-free protein synthetic system used in the continuous transcriptional/translational non-coupling cell-free protein synthetic means according to any of the above 15 to 22 is a system utilizing a wheat germ extract and, in the said wheat germ extract, contamination of endosperm components is substantially removed.
24. A method for the co-expression of plural proteins using a cell-free protein synthetic means, characterized in that, two or more mRNA's which are translational templates in a cell-free protein synthetic means are used and each mRNA is separately pre-incubated in a cell-free protein synthetic reaction solution followed by mixing to conduct a cell-free protein synthesis.
25. The method for the co-expression of plural proteins according to the above 24, wherein the mRNA used is an mRNA which is synthesized by any of the synthetic methods for RNA according to the above 8 to 11.
26. The method for the co-expression of plural proteins according to the above 24 or 25, wherein the cell-free protein synthetic means is a cell-free protein synthetic means using a wheat germ extract and contamination of the endosperm components in the said wheat germ extract is substantially removed.
27. A wheat germ material for the cell-free protein synthesis comprising only yellow germ where germ having small injured area having white color and germ having brown or black color are removed.
28. A wheat germ extract which is manufactured from the wheat germ material according to the above 27.
29. The cell-free protein synthetic means according to the above 14, wherein the wheat germ extract where contamination of endosperm components is substantially removed is the wheat germ extract according to the above 28.
30. The continuous transcriptional/translational non-coupling cell-free protein synthetic means according to the above 23, wherein the wheat germ extract where contamination of endosperm components is substantially removed is the wheat germ extract according to the above 28.
31. The method for the co-expression of plural proteins according to the above 26, wherein the wheat germ extract where contamination of endosperm components is substantially removed is the wheat germ extract according to the above 28.
32. A cell-free protein synthetic means, characterized in that, in a cell-free protein synthetic system using a wheat germ extract, the cell-free protein synthetic reaction is carried out under such a condition that a reaction solution containing the wheat germ extract is not stirred under a static condition.
33. The cell-free protein synthetic means according to the above 14 or 29, wherein the cell-free protein synthetic reaction is carried out under such a condition that a reaction solution containing the wheat germ extract is not stirred under a static condition.
34. The continuous transcriptional/translational non-coupling cell-free protein synthetic means according to the above 23 or 30, wherein the cell-free protein synthetic reaction is carried out under such a condition that a reaction solution containing the wheat germ extract is not stirred under a static condition.
35. The method for the co-expression of plural proteins according to the above 26 or 31, wherein the cell-free protein synthetic reaction is carried out under such a condition that a reaction solution containing the wheat germ extract is not stirred under a static condition.
36. A method for the test of the relation between gene polymorphism and gene function, characterized in that, one means/method selected from the cell-free protein synthetic means according to the above 12∼14, 29 and 33, the continuous transcriptional/translational non-coupling cell-free protein synthetic means according to the above 15∼23, 30 and 34 and the method for the co-expression of plural proteins according to the above 24∼26, 31 and 35 is utilized.
37. A method for the screening of the gene function, characterized in that, one means/method selected from the cell-free protein synthetic means according to the above 12∼14, 29 and 33, the continuous transcriptional/translational non-coupling cell-free protein synthetic means according to the above 15∼23, 30 and 34 and the method for the co-expression of plural proteins according to the above 24∼26, 31 and 35 is utilized.
38. A method for the synthesis of protein using one means/method selected from the cell-free protein synthetic means according to the above 14, 29 and 33, the continuous transcriptional/translational non-coupling cell-free protein synthetic means according to the above 23, 30 and 34 and the method for the co-expression of plural proteins according to the above 26, 31 and 35, characterized in that, protein is synthesized from gene derived from mammal utilizing a wheat germ extract.

### Brief Description of the Drawings

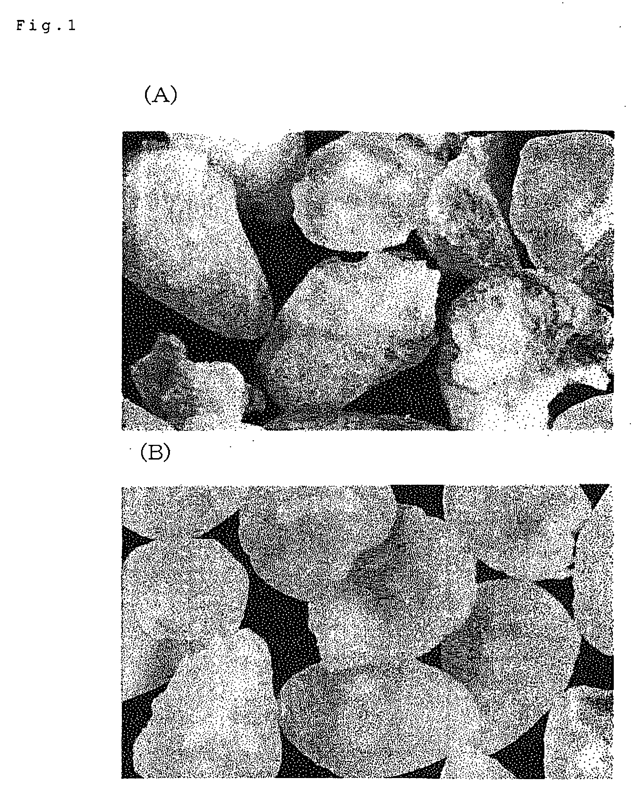

Fig. 1: This is a drawing which illustrates the criteria for the selection by color and shape in the selection of wheat germ used for the preparation of a cell extract for a cell-free protein synthesis. In Fig. 1, the left one (A) shows germ having a small injured area having white color and germ in brown and black colors while the right one (B) shows a wheat germ material for a cell-free protein synthesis comprising yellow germ only.

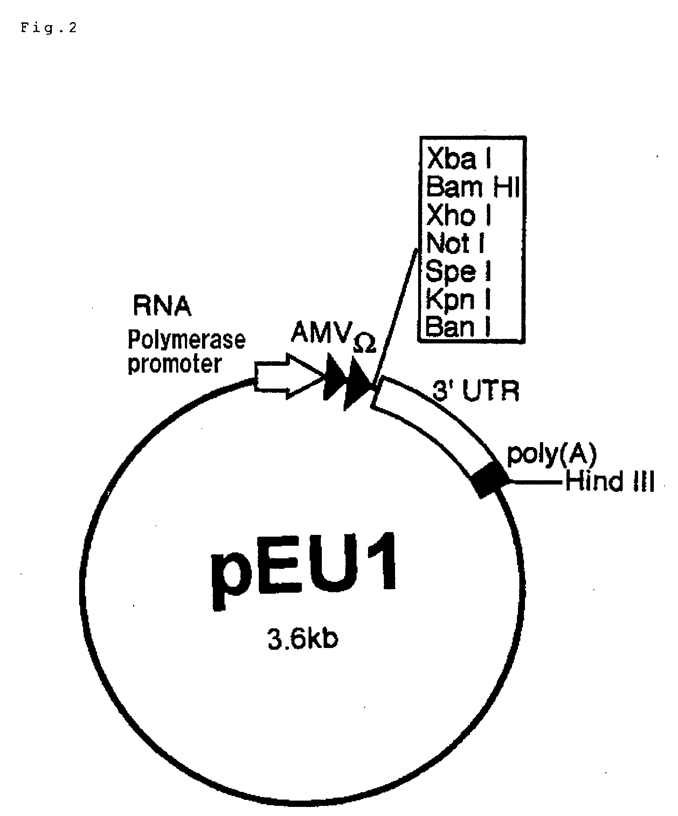

Fig. 2: This is a scheme of a plasmid (pEU1) for synthesis of template molecule for a cell-free protein synthesis having a broad applicability and a highly efficient translational template function.

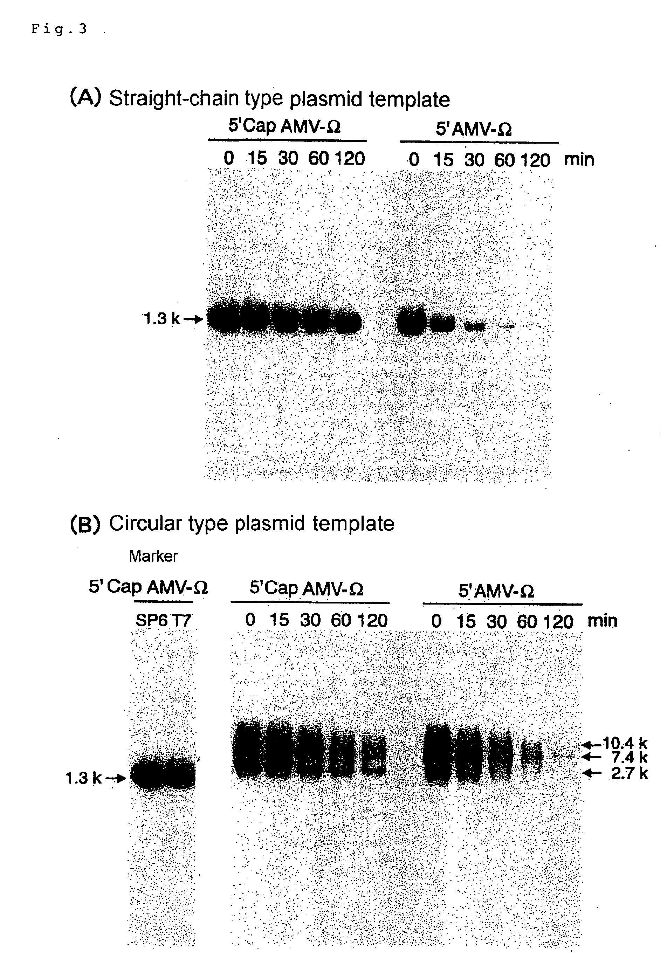

Fig. 3: This is a drawing where mRNA in which 3'-terminal non-translational sequence is linked to mRNA having an AMV-Ω sequence at 5'-terminal is designed and synthesized and stability of those template molecules in a cell-free protein synthetic reaction utilizing a wheat germ extract is illustrated. In Fig. 3, (A) and (B) show the stabilities of the mRNA synthesized using a straight-chain type plasmid and a circular type plasmid, respectively.

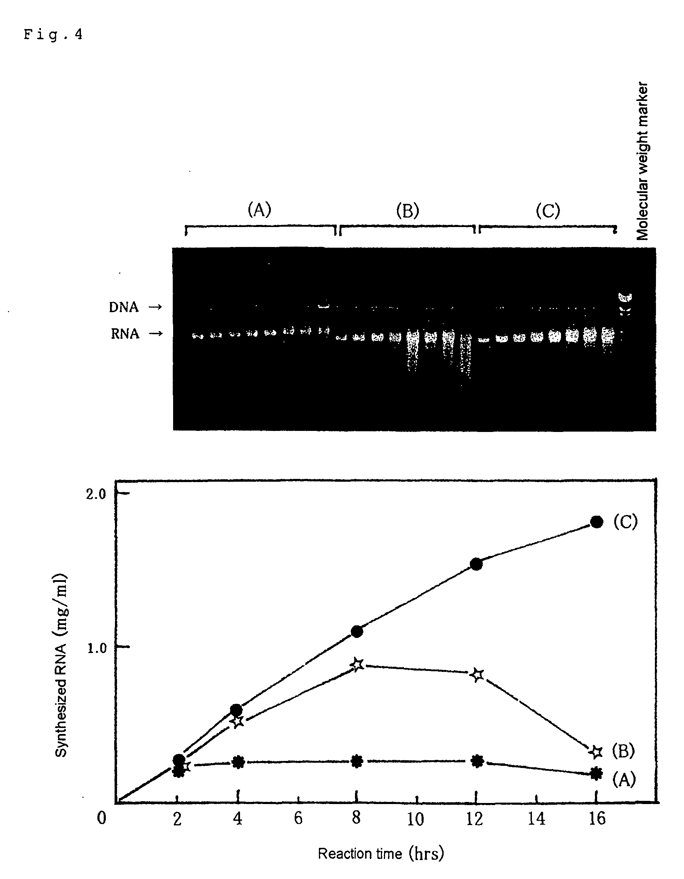

Fig. 4: This is a drawing which illustrates the result of comparison of synthetic efficiency of the transcription reaction between the conventional batch reaction (reaction method *in vitro*) and the novel continuous reaction method utilizing a dialysis membrane. The upper drawing is that the transcribed product during the course of the reaction is separated by an agarose gel electrophoresis and stained with ethidium bromide. The lower drawing shows the changes of the amount of the synthesized mRNA with elapse of time which is calculated from the absorbance value at A260 nm after purification and the ratio of fluorescence intensity of mRNA on the gel to the fluorescence intensity of the template DNA that is an internal standard. In the drawing, (A), (B) and (C) show the results of a reaction method *in vitro,* a dialysis membrane to which no serum albumin coating is applied and a dialysis membrane coated with serum albumin are used, respectively.

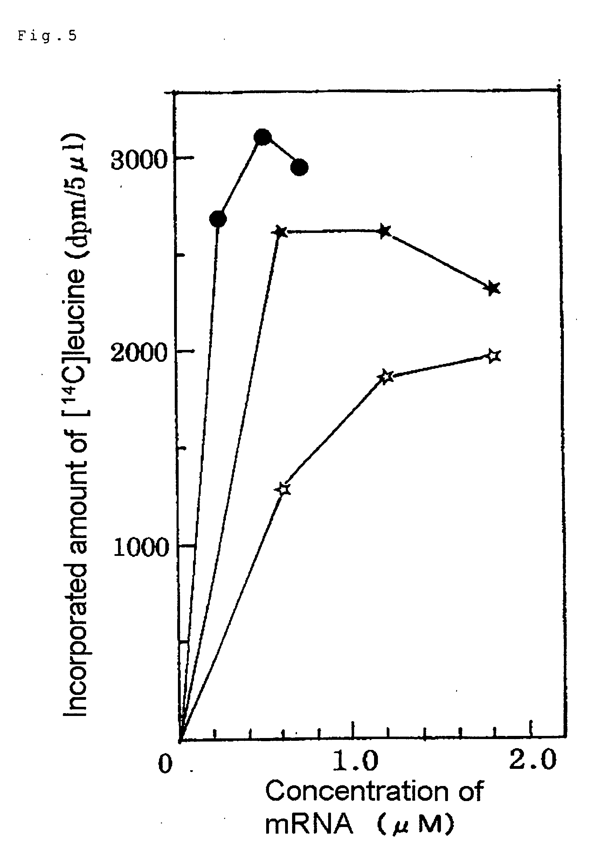

Fig. 5: This is a drawing which compares the template activity of various mRNA's synthesized by a dialytic method in a wheat germ cell-free protein synthetic system of a batch type. In the drawing, 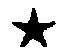 - 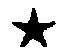 shows dhfr mRNA where an AMV-Ω sequence is linked to 5' -terminal and poly (A) 100 and 3'-UTR derived from dhfr gene of *Escherichia coli* is linked to 3'-terminal which is synthesized from a circular type plasmid (being un-incised by restriction enzyme); 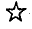 - 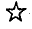 shows the same mRNA synthesized from a straight-chain type plasmid (being incised by restriction enzyme) ; and ●-● shows mRNA having a CAP structure at 5'-terminal synthesized by using a straight-chain type plasmid as a template. The ordinate shows a radioactivity (dpm/5 µl) incorporated in protein while the abscissa shows the concentration of mRNA in the reaction solution.

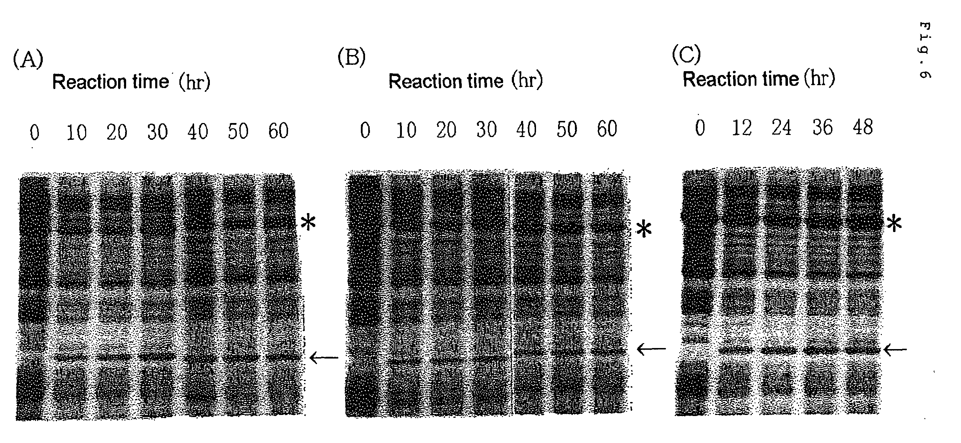

Fig. 6: This drawing shows the result of the continuous cell-free protein synthesis using a dialysis membrane by the use of mRNA molecule species having a 5'-AMV-Ω structure synthesized using straight-chain type and circular type plasmids as templates. (A) and (B) of Fig. 6 show the result of protein synthesis in the case where mRNA is synthesized using circular type and straight-chain type plasmids, respectively, and used as translational templates. (C) of Fig. 6 shows the result of synthesis of protein where mRNA is synthesized in a microspin column using a circular type plasmid as a template and purified by centrifugation utilizing the said reaction vessel, the filter collecting the mRNA is taken out from the microspin column and the filter is added to an inner area of dialysis membrane filled with a protein synthesis reaction solution. In the drawing, an arrow means dhfr protein which is a translation product while a star means creatine kinase in the reaction solution.

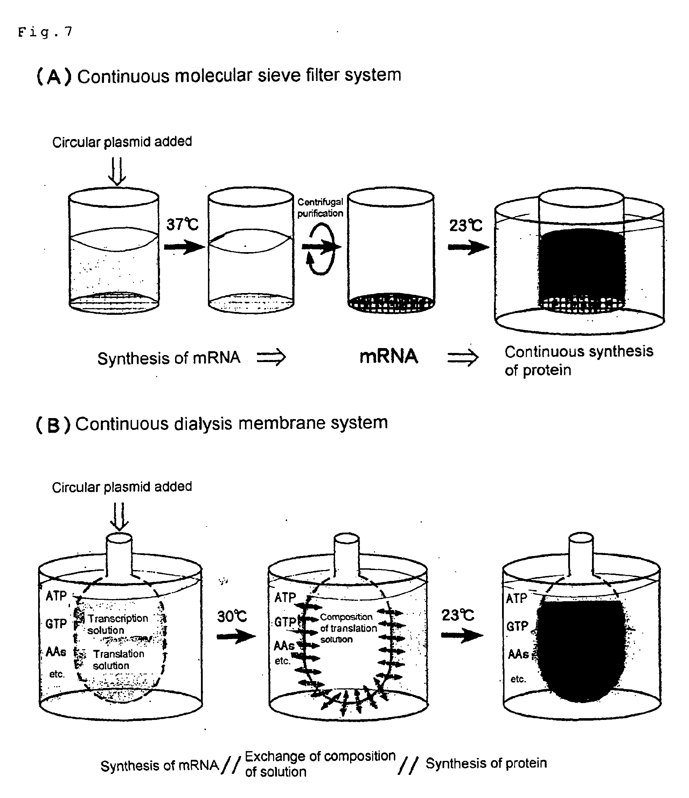

Fig. 7: This drawing illustrates a continuous and united transcriptional/translational non-coupling system comprising a continuous mRNA synthesis and a continuous cell-free protein synthesis. (A) and (B) of Fig. 7 illustrate a cell-free protein synthetic method using pure mRNA collected on the filter as a template and a continuous transcriptional/translational non-coupling cell-free protein synthesis method utilizing a dialysis membrane, respectively.

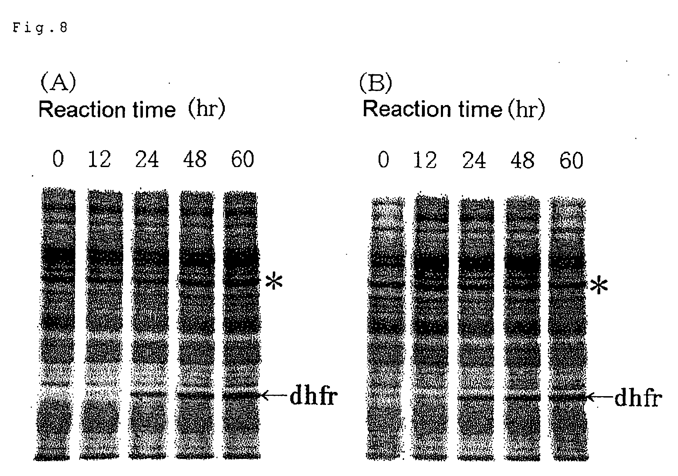

Fig. 8: This shows the result of protein synthesis by a continuous and united transcriptional/translational non-coupling system comprising the continuous RNA synthesis and the continuous cell-free protein synthesis. Fig. 8(A) shows the result using a continuous transcriptional/translational cell-free protein synthetic method utilizing a dialysis membrane and Fig. 8(B) shows the result of a method using pure mRNA collected on the filter as a template. In the drawing, an arrow means dhfr which is a translation product and a star means creatine kinase in the reaction solution.

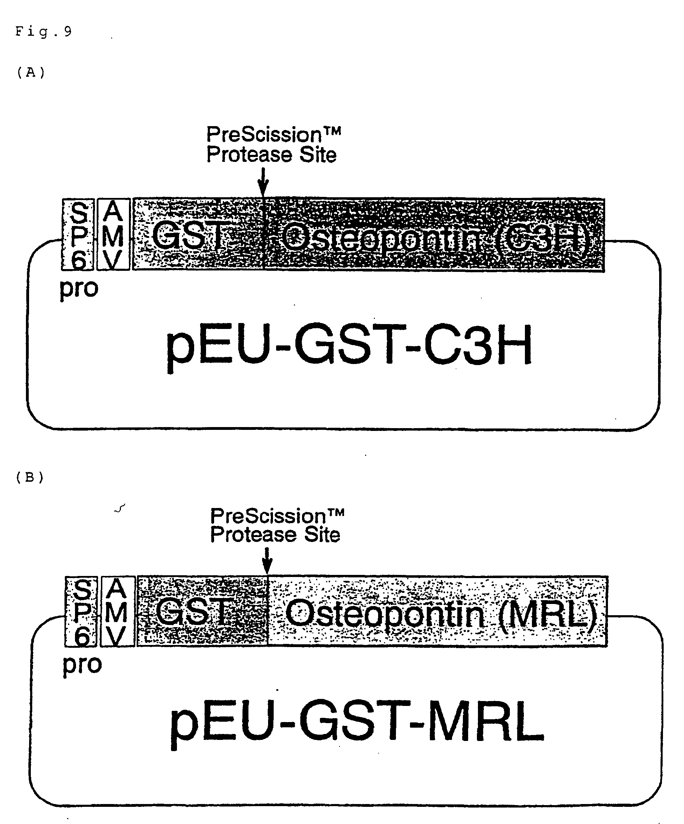

Fig. 9: This is a scheme showing the plasmid for. a cell-free protein synthesis of osteopontin which is a fusion type with GST. Fig. 9(A) shows pEU-GST-C3H which is a plasmid containing ORF coding for osteopontin derived from C3H mouse fused with GST and Fig. 9(B) shows pEU-GST-MRL which is a plasmid containing ORF coding for osteopontin derived from MRL mouse fused with GST.

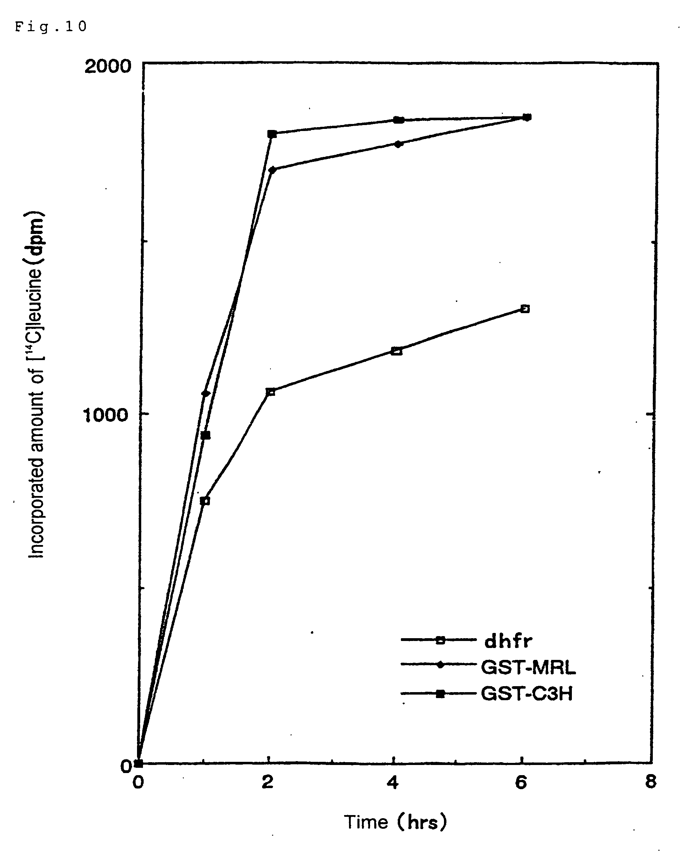

Fig. 10: This shows the result of synthesis of osteopontin of a fused type by means of a cell-free protein synthesis of a batch type using a wheat germ extract.
Fig. 11: This is an autoradiogram where molecular weight of a synthesized osteopontin of a fused type is confirmed. In the drawing, M is a molecular weight marker.

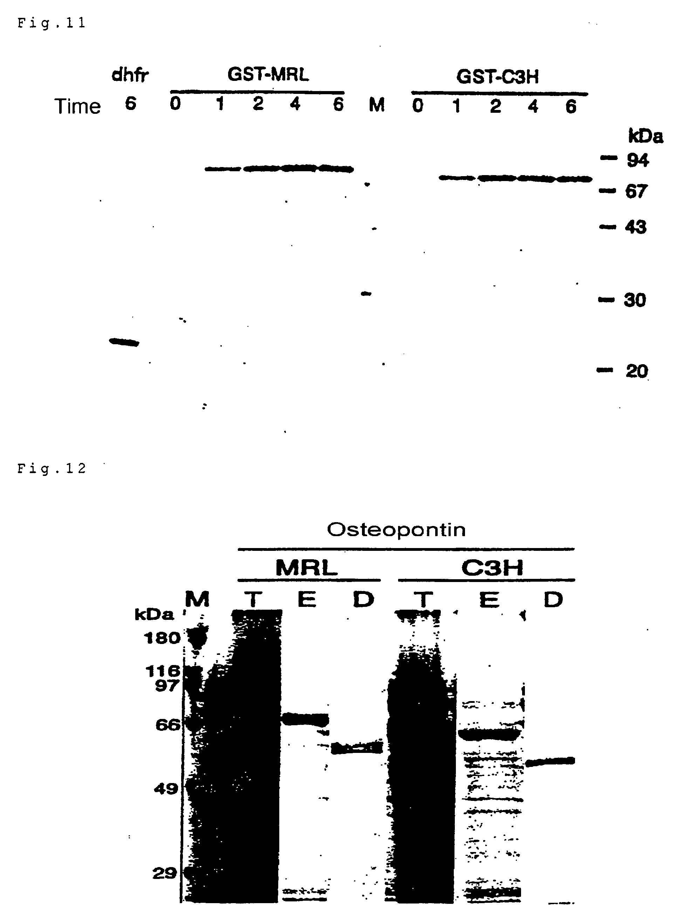

Fig. 12: This is a drawing where osteopontin of a fused type obtained by a continuous cell-free protein synthesis by a dialytic method using a wheat germ extract is confirmed by means of staining with Coomassie Blue after an SDS-polyacrylamide gel electrophoresis. In the drawing, M is a molecular weight marker. Lane T is a separated and stained pattern of total protein including the synthesized fused protein (GST-OPN), lane E is that where the said synthesized fused protein is purified and lane D is an OPN protein of a mature type.
Fig. 13: This shows the result of co-expression of five kinds of proteins by a cell-free protein synthesis. Fig. 13 (A) shows the result where each protein is separately synthesized and the result where mRNA of each protein is mixed to conduct a cell-free protein synthesis. In the drawing, dhfr means dihydrofolate reductase, GST means glutathione-S-transferase, GFP means green fluorescent protein, G-OPN means GST-osteopontin fused protein and LUC means luciferase. Further, -mRNA means the result where a cell-free protein synthetic reaction is carried out without the use of mRNA. MIX is a conventional method and is a result where each mRNA is mixed followed by carrying out a cell-free protein synthesis. A star shows each of the synthesized protein. Fig. 13 (B) shows the result where each of the above-mentioned five kinds of mRNA's is separately incubated and then mixed to carry out a cell-free protein synthesis. M is a molecular weight marker.
Fig. 14: This shows that a cell-free protein synthetic reaction under a static condition without stirring [Fig. 14(A)] has better synthetic efficiency than the case where the reaction is carried out with stirring [Fig. 14(b)]. In the drawing, an arrow shows the synthesized GEP.

### Best Mode for Carrying Out the Invention

The present inventor has specified the causes whereby the cell-free protein synthesis system becomes unstable and, on the basis of such a finding, he has developed a means for the preparation of cell or tissue extract which is stable and has high synthetic efficiency and a means for a highly efficient cell-free protein synthesis (PCT/JP99/04088). Hereinafter, the term "means" stands for a method and/or a medium used for achieving the object.

In order to accomplish the practical cell-free protein synthetic means, the following technique has been further established in the present invention. Thus, there have been established the development of non-translational sequences of 5'-terminal and 3' -terminal of mRNA which significantly promote the translation efficiency to any of mRNA species; the construction of plasmid having a broad applicability for the synthesis of mRNA being incorporated with the said non-translational sequence and having a high translation efficiency (hereinafter, may be referred to as highly effective translation cassette plasmid); the development of simple and convenient technique for synthesis in large quantities and purification of mRNA using the said cassette plasmid as a template; the development of a simple and convenient system for carrying out a continuous transcriptional/translational non-coupling protein synthesis wherein a continuous RNA synthesis utilizing a ultrafilter membrane and a continuous cell-free protein synthesis using a dialysis membrane are united; the method in which the highly efficient translation cassette plasmid incorporated with the aimed protein gene is not cleaved by a restriction enzyme but the original circular type plasmid is added to a cell-free protein synthetic system whereby mRNA which is stable and has a high translational template activity is continuously synthesized in a good efficiency so that, in the same reaction vessel, a continuous cell-free protein synthesis is made possible in a non-coupling manner with transcription; the method for the co-expression of plural proteins using a cell-free protein synthetic means; etc.

As hereunder in the present invention, the cell extract derived from wheat germ, *Escherichia coli,* reticulocyte of rabbit, etc. may be used for a cell-free protein synthetic means although a wheat germ extract is preferred and, particularly preferably, a wheat germ extract wherefrom contamination of endosperm components is substantially removed is used. With regard to the material for the said wheat germ extract, the particularly preferred one is a preparation made from a material which is a wheat germ comprising yellow germ only [Fig. 1(B)] obtained by a method which will be mentioned later in Example 1 where germ having a white small injured part and germ having brown or black color [Fig. 1(A)] are removed.

When a cell-free protein synthetic means is carried out in the present invention using a wheat germ extract, it is preferred that the protein synthesis reaction is carried out under a static condition where the reaction solution is not stirred.

### (Construction of 5'-terminal and 3'-terminal of mRNA having a high translation efficiency)

### 5'-Terminal structure:

Construction of the highly efficient protein translational template molecule according to the present invention is carried out in such a manner that, on the basis of a finding concerning the 5'-terminal non-translational sequence obtained from the investigation for a protein expression system using living cells, an alfalfa mosaic virus (AMV) leader sequence, an omega sequence existing in a 5'-terminal non-translational sequence of tobacco mosaic virus (TMV) RNA or a base sequence described in SEQ ID NO: 1 or SEQ ID NO: 2 of the Sequence Listing obtained by modifying the above is used as a 5'-untranslated region (5'-UTR) (free text of the Sequence Listing) and introduced into 5'-terminal of the aimed mRNA in the base sequence of a protein translational template molecule (mRNA) used in a cell-free protein synthetic system. The AMV leader sequence and the TMV omega sequence will be shown in Table 1 while the three base sequences obtained by modifying the above (SEQ ID NO: 1 to NO: 3 of the Sequence Listing) will be shown in Table 2.

The base sequence which is to be introduced may be an alfalfa mosaic virus (AMV) leader sequence, an omega sequence existing in the 5'-terminal non-translational region of tobacco mosaic virus (TMV), a base sequence described in SEQ ID NO: 1, SEQ ID NO: 2 or SEQ ID NO: 3 of the Sequence Listing or a base sequence having a homology of 70% or more to those sequences and being able to give about 70% or more translation initiation activity as compared with the translation initiation activity of mRNA wherein CAP structure exists.

The said base sequences are also possible to be introduced either solely or in plural, further in combination with the base sequence or structure related to translation initiation such as known CAP structure.

### 3'-Terminal structure:

It has been known that, in the 3'-terminal of eukaryotic RNA, from several tens to about 200 polyadenylic acid structures [poly(A)] are present and participate in promotion of translation initiation activity and in stabilization of RNA. The present inventor has synthesized mRNA by adding 50∼150 or, preferably, 100 polyadenylic acid structures to the 3' -terminal of template DNA of RNA and confirmed that the said molecule has a high template activity.

In order to investigate the further stabilization of mRNA on the basis of those results, the present inventor has introduced a long non-translational RNA chain into a downstream of translation termination codon and found that stability of mRNA significantly increased. With regard to a method for the introduction of this long-chain non-translational RNA, plasmid was not cleaved by a restriction enzyme before the transcription reaction to give a straight-chain type but the original circular type was used in the transcription reaction as a template. In that case, no special transcription termination site was inserted to the used plasmid and, therefore, mRNA which is a transcribed product was synthesized as mRNA having various molecular sizes.

Incidentally, the 3'-terminal non-translational sequence which is the long-chain non-translational RNA may be about 300 bases or more or, preferably, about 500 bases or more and the said base sequence may not be a special one. Preferably, it is about 500 to about 1,500 bases and, most preferably, about 700 bases.

As an examples of the present invention, there was adopted a method of introducing the non-translational sequence derived from viral RNA to 5'-terminal and/or 3'-terminal of the gene by a gene engineering means utilizing gene of dihydrofolate reductase (dhfr) of *Escherichia coli* cloned into the plasmid (Baranov, V. I., et al., (1989), *Gene,* 84, 463-466). Thus, as one of them, a novel structure of mRNA requiring no existence of CAP structure and having a high translation initiation activity was constructed by introducing AMV leader sequence which is 5'-terminal non-translational sequence of AMV mRNA, Ω structure of 5'-terminal non-translational sequence of TMV mRNA or base sequence described in SEQ ID NO: 1, SEQ ID NO: 2 or SEQ ID NO: 3 of the Sequence Listing in plural in a combined manner in series into 5'-terminal thereof or by introduction of a long non-translational sequence (UTR) and/or 100 polyadenylic acid structures into 3'-terminal thereof. Such sequences and structures potentiate the translation activity of the said mRNA even when each of them is introduced into mRNA solely. However, the structure of mRNA having a high translation activity is in such a case that, as shown in Table 1 of Example 1, AMV-Ω-base sequence being translated for protein -3'-terminal non-translational sequence-poly(A)-3'-terminal non translational sequence derived from the plasmid is synthesized by the use of a circular type plasmid as a transcriptional template or, in place of the AMV-Ω, a base sequence described in SEQ ID NO: 1, SEQ ID NO: 2 or SEQ ID NO: 3 of the Sequence Listing is similarly introduced and constructed (Table 2 and Table 3).

As hereinabove, it has been succeeded in designing and synthesizing the mRNA having a high template activity when novel 5'-terminal and 3'-terminal structures are linked to mRNA and it has been proved that the product shows a high translation efficiency in a cell-free protein synthetic system utilizing a wheat germ extract. The above-mentioned modifying means for a translational template molecule according to the present invention can be utilized for the synthesis of RNA having a high template activity and for a cell-free protein synthetic means.

### (Construction of plasmid having a broad applicability for the synthesis of mRNA having a high translation efficiency)

The above-mentioned finding was obtained from the result of the experiment using dhfr gene as a model and, in order to make it commonly utilizable for various mRNA syntheses, there was constructed novel plasmid for an *in vitro* RNA synthesis having a broad applicability where plural cloning sites are inserted. It goes without saying that, in a template DNA for mRNA transcription to be incorporated into the said plasmid as an ORF, it is possible to link a DNA encoding a tag such as GST (glutathione-S-transferase). With regard to the tag, although that which has been known *per se* may be used, it is also possible upon necessity to use commercially available ones such as histidine tag (His-tag), FLAG, Xpress, hemagglutinin epitope (HA), Myc epitope, T7 epitope, HSV epitope, maltose-linked protein (MBP), thioredoxin (TRX), β-galactosidase (βgal, LacZ), etc. With regard to the said plasmid, not only that which is previously cleaved into a straight-chain type by a restriction enzyme but also a circular type without cleaving may be used as a template for the transcription reaction whereupon it is possible to synthesize the mRNA where a long non-translational RNA chain is introduced at the 3'-terminal side of mRNA. It has been further confirmed that this transcription product (mRNA) has a stable and high translational template function in a cell-free protein synthetic system utilizing a wheat germ extract.

The plasmid having a broad applicability according to the present invention is a plasmid utilized as a template in a cell-free protein synthetic wherein
① a base sequence carrying a promoter function,
② a base sequence at the downstream thereof which is to be a transcriptional template for at least
   an alfalfa mosaic virus (AMV)leader sequence,
   an Ω sequence that exists in a 5'-terminal non-translational sequence of tobacco mosaic virus (TMV),
   a base sequence described in SEQ ID NO: 1, SEQ ID NO: 2 or SEQ ID NO: 3 of the Sequence Listing or
   a base sequence having 70% or more homology to those base sequences and being able to give mRNA which has about 70% or more translation initiation activity as compared with the translation initiation activity of mRNA where a CAP structure is present,
③ plural restriction enzyme cleavage sites at the region corresponding to the downstream thereof and
④ at least 3'-terminal non-translational sequence at the region corresponding to the downstream of the termination codon of ORF
   are aligned. When the plasmid of the present invention is used in a circular type as it is, no special transcription termination site is inserted into the said plasmid as mentioned already and, therefore, the mRNA which is a transcription product is synthesized as mRNA having various molecular sizes. It is also possible that a sequence carrying a terminator function regulating the transcription termination site is aligned at the upstream of the promoter region so that the molecular size of the transcription product is made constant.

With regard to the plasmid utilized here, there is no particular limitation so far as it is able to be a template for mRNA utilized for a cell-free protein synthetic system using a cell extract such as wheat germ, *Escherichia coli* and reticulocyte of rabbit. It is however preferred that the size of the plasmid itself is 3 Kb or more. With regard to a promoter, there is also no particular limitation so far as it is able to achieve the function for the synthesis of mRNA in the said cell-free protein synthetic system and there may be exemplified SP6 promoter and T7 promoter. Plural sites cleaved by restriction enzyme are the means that, by utilizing the sites, desired gene is inserted into the plasmid as a template for the synthesis of mRNA.

The plasmid of the present invention obtained as such can be used for the means of RNA synthesis and a cell-free protein synthesis, etc.

### (Establishment of simple means for mass synthesis and purification of mRNA)

The present inventor has invented a new technique for the following continuous RNA synthesis whereby the problems in the conventional RNA synthetic methods are solved. Thus, a spin column equipped with an ultrafiltration filter is prepared as a reaction vessel and, in the said reaction vessel, there is filled a transcription reaction solution comprising template DNA and 4 kinds of substrate ribonucleoside-5'-triphosphates and, if necessary, CAP molecule, RNA polymerase, spermidine, magnesium ion, appropriate buffer, etc. A solution separately prepared by the excluding template DNA and RNA polymerase from the above-mentioned transcription reaction solution is used as an external solution for dialysis and a dialytic continuous synthesis of RNA is carried out. In this RNA synthetic method, it is possible to continuously supply the substrate and to continuously exclude pyrophosphoric acid, etc. resulted as by-products from the reaction system and, since the reaction does not reach the chemical equilibrium for long time, synthesis of RNA continues as long as 12 hours or more whereby 5-fold more of RNA as compared with the conventional method can be obtained.

With regard to the molecular weight cutting size of the ultrafiltration filter, there is no particular limitation so far as it is a pore size by which separation of the synthesized RNA from the low-molecular substances such as substrate and by-products is possible. It is however preferred to use a filter having a cutting molecular weight of 5,000∼100,000. Pore size of such a filter is 10∼50 Å.

In the RNA synthetic method of the present invention, it is possible to utilize the above-mentioned modifying means for translational template molecule of the present invention and it is also possible to use the above-mentioned plasmid of the present invention.

It has also been firstly found in the present invention for the priority of applying a protein coating on the surface contacting the reaction solution of the reaction vessel carrying out the mRNA synthesis, at least on the surface participating in a dialyzing means or a molecular sieving means such as a dialysis membrane or ultrafiltration filter, in a synthetic method for mRNA in a cell-free protein synthetic means. Thus, a mRNA transcription efficiency can be increased by a coating treatment using protein and degradation of mRNA can be prevented as well. With regard to the protein used for the coating, albumin is exemplified although it is not limited thereto only. Further, it is not necessary that the protein used for coating is particularly fixed to the surface participating in the dialyzing means or a molecular sieving means but the effect may be achieved by washing with a protein-containing solution for several times although this is not a limitation. A method of washing with a protein-containing solution for several times is simple and preferred. Concentration of the protein-containing solution is not particularly limited but a saturated concentration for dissolving is sufficient and lower than that is acceptable as well.

The point where the RNA synthetic method of the present invention is better than the conventional methods is that purification of the synthesized RNA is simple. Thus, in the present method, the reaction vessel is just centrifuged at low speed after synthesis of RNA whereby the low-molecular substances are removed from the pores of the ultrafiltration filter while RNA molecules are collected on the ultrafiltration filter in the reaction vessel. When addition of an appropriate solution such as water and the succeeding centrifugation are repeated for several times, RNA having a very high purity can be easily isolated and recovered without conducting an operation such as treatment with phenol.

As such, when the modifying means for template molecule of the present invention or the plasmid of the present invention is utilized and, the continuous RNA synthetic method of the present invention is used, it is now possible to manufacture the RNA more efficiently than before. The present method is also applicable for the synthesis of mRNA and for the synthesis of other RNA's in general.

The mRNA obtained by the RNA synthetic method of the present invention is utilizable to a cell-free protein synthetic means.

### (Cell-free protein synthetic means)

In the present invention, the above-mentioned modifying method for translational template molecule is utilized or a cell-free protein synthetic means utilizing the above-mentioned plasmid is included as well. The cell-free protein synthetic means of the present invention uses the mRNA having a high translation efficiency of the present invention or using the plasmid of the present invention and, therefore, a higher protein synthetic efficiency than in the conventional method is achieved. In addition, the cell-free protein synthetic means of the present invention can be utilized in a continuous transcriptional/translational non-coupling cell-free protein synthetic means of the present invention which will be mentioned later.

### (Unified system of a continuous RNA synthesis and a continuous cell-free protein synthesis)

Further, in maintaining the high synthetic efficiency and in simplifying the operation in a cell-free protein synthetic means, there have been established the following two practical systems where m-RNA synthesis and protein synthesis are unified in which, in carrying out the cell-free protein synthetic reaction, plasmid containing the aimed protein gene is added to the reaction system whereby a sufficient amount of protein can be synthesized. Any of those two systems is a continuous system where mRNA is synthesized firstly and then protein synthesis is carried out in the same reaction vessel and is a transcriptional/translational non-coupling system. This system is useful for the development of a fully automated cell-free protein synthetic apparatus as well.

### (System utilizing a dialysis membrane)

Fig. 7 (B) shows a scheme of a continuous transcriptional/translational non-coupling cell-free synthetic means of the present invention where a continuous RNA synthesis utilizing the dialysis membrane and a continuous cell-free protein synthesis are unified.

Firstly, an aimed gene is integrated into the plasmid for the mRNA synthesis including the above-mentioned 5'-terminal and 3'-terminal non-translational sequences having a high translation activity and that is added in a circular type as it is to a transcription reaction solution and subjected to a preliminary reaction at 23∼37°C or, preferably, at 30°C for about 5 minutes or, preferably, for 10 minutes. Although this preliminary reaction step may be omitted if desired, it is advantageous to carry out the said reaction because, in this preliminary reaction step, RNA polymerase binds to a promoter region of a template DNA and the transcription initiation reaction efficiently proceeds whereby the synthesized amount of mRNA in the next heating step increases and, therefore, the synthesized amount of protein in the translation reaction stage increases. After that, the transcription reaction solution and the translation reaction solution are mixed. The mixture is transferred to a dialysis membrane tube and dipped in a container filled with a previously-prepared external solution for dialysis for the synthesis of protein. The reaction is carried out under a static condition at 30°C for about 3 hours whereupon, at first, mRNA is mainly synthesized.

In the next step, the reaction temperature is lowered to 23°C and the reaction under dialysis is maintained under a static condition. In this stage, concentrations of low-molecular substances in the inner area of dialysis membrane particularly ATP, GTP and magnesium ion which are set at high corresponding to the optimum concentration of mRNA synthesis lower together with the progress of dialysis, the concentration comes near the concentration of the external solution for dialysis comprising a solution for protein synthesis and, soon, the inner area of the dialysis membrane is converted to a composition of solution for protein synthesis.

As a result thereof, a protein synthesis reaction is promoted and its reaction rate becomes maximum whereupon protein is mostly synthesized. This cell-free protein synthetic reaction continues for 60 hours or longer and, in the case of a dhfr synthesis, it is possible to obtain about 4 mg of protein per ml of the reaction solution.

In the system utilizing the dialysis membrane, any of plasmids of circular type and straight-chain type may be used. However, when the synthetic yield and the cost are taken into consideration, it is preferred to use a circular type plasmid. Further, in any of the conditions of presence and absence of CAP, the present system can be carried out but, since the CAP itself used for the mRNA synthesis strongly binds to and inhibits a factor which is necessary for the protein synthesis and the efficiency of the protein synthesis lowers, it is preferred to carry out that in the absence of CAP.

### (System utilizing an ultrafiltration filer)

A scheme of the continuous transcriptional/translational non-coupling cell-free synthetic means according to the present invention where a continuous RNA synthesis utilizing an ultrafilter membrane and a continuous cell-free protein synthesis are united is shown in Fig. 7(A).

mRNA is synthesized by a dialytic method using a spin column equipped with an ultrafilter membrane using a plasmid containing the above-mentioned 5'-terminal and 3'-terminal non-translational sequence having a high translation activity as a template. In that case, it is preferred that the plasmid which is a template for the RNA synthesis is used in a circular form as it is. It is incubated at 30∼37°C and the reaction time is determined depending upon the necessary synthetic amount of RNA and, usually, it may be about 3∼5 hours. The spin column equipped with an ultrafilter membrane used here is the same as that use in the synthetic method for RNA of the present invention.

After the reaction, the spin column which is a reaction vessel is centrifuged and washed whereupon the purified mRNA is collected on a filter. Then a solution for a cell-free protein synthesis containing a cell extract such as a wheat germ extract is added to the said spin column and dipped in a container filled with an external solution for the translation reaction, a cell-free protein synthetic reaction is started at 23°C and, when a wheat germ extract is used as a cell extract, the reaction is carried out under the static condition.

The synthetic yield of protein obtained by the present invention is nearly as same as that in the case of the above-mentioned method using a dialysis tube. It is also possible to synthesize the protein when the mRNA on the filter of the spin column is poured together with the filter into a dialysis tube in the above-mentioned system utilizing a dialysis membrane. Further, when the present means is used, mRNA having a CAP structure can be also subjected to an efficient translation reaction after its synthesis and purification in an entirely same manner.

In a system utilizing an ultrafilter membrane, any of plasmid of circular and straight-chain types may be used and the present system can be carried out under any of the conditions of presence and absence of CAP. Although the synthetic yield of protein is higher when mRNA having a CAP structure at the terminal is used, there is no big different in the synthetic yield of mRNA having a CAP structure at the 5'-terminal synthesized from a straight-chain type plasmid and that of mRNA synthesized from a circular type plasmid in the absence of CAP and, when the cost is taken into consideration, it is preferred to use a circular type plasmid in the absence of CAP.

In the above-mentioned present two systems, with regard to one or more element(s) at least selected from mRNA which is to be a translational template for the protein synthetic reaction, enzyme of energy regeneration system, substrate and energy source, there may be introduced a means where it/they is/are additionally added either upon necessity or continuously after starting the reaction. About that, very small amount may be added continuously or intermittently. Further, in the present system, a treatment wherein a substance which is necessary for the above-mentioned cell-free protein synthetic reaction is exchanged after the start of the said synthetic reaction and during the reaction may be introduced so as to maintain the efficiency of the said synthetic reaction. For example, a means wherein a desired amount of the external solution for dialysis comprising a solution for a cell-free protein synthesis is exchanged either continuously or intermittently may be introduced. Further, for additional addition or exchange of the necessary substance for the above-mentioned cell-free protein synthetic reaction, the present system may have a means for preservation of that. Furthermore, the present invention may have a discharging means for excluding the by-products in the cell-free protein synthetic reaction from the said synthetic reaction. With regard to the means for such addition, preservation, exchange and/or discharge, it is possible that one or more thereof is/are selected, combined and introduced.

Still further, in the above-mentioned present system, when a protein coating is previously applied to at least the surface participating in the dialyzing means or molecular sieving means, such as a dialysis membrane or a ultrafilter membrane, among the surfaces contacting the reaction solution of the reaction vessel where the synthesis is carried out efficiency of the protein synthesis is further improved. Thus, as a result of a coating treatment using protein, it is possible to enhance the efficiency of the mRNA transcription and the efficiency of the succeeding protein translation. With regard to the protein used for the coating, albumin is exemplified but that is not a limitation. Further, with regard to the protein used for the coating, it is not necessary that it is specifically fixed to the surface participating in the dialyzing means or the molecular sieving means and, although an effect is achieved even when washed with a solution containing protein for several times and that is simple and convenient, the present invention is not limited thereto. For example, when a dialysis membrane is coated with albumin, a mixed solution of water and albumin may be used as an internal solution while water is used as an external solution and dialysis may be carried out for several hours. It is of course possible that the compositions of the internal and external solutions are reversed. Concentration of the protein-containing solution is not particularly limited and a saturated concentration for dissolving is sufficient or less than that will do as well.

### (Method for co-expression of plural proteins utilizing a cell-free protein synthetic system)

The present invention further provides a method for the co-expression of plural proteins utilizing a cell-free protein synthetic system. The method for the co-expression of plural proteins according to the present invention is characterized in that, firstly, each mRNA of the aimed plural proteins is separately subjected to a pre-incubation in a reaction solution for a cell-free protein synthesis, then they are mixed and a cell-free protein synthesis is carried out. The mRNA used here may be the mRNA which is synthesized by the above-mentioned RNA synthetic method of the present invention. Appropriate time for the pre-incubation is from 5 minutes to 12 hours, preferably from 30 minutes to 3 hours and, particularly preferably, 3 hours. When the pre-incubation is shorter than 5 minutes, co-expression of plural proteins is not noted while, when it is longer than 13 hours, partial insolubilization of the product takes place and they are not preferred. A cell-free protein synthesis after the pre-incubation may be carried out by means of the conventional cell-free protein synthetic method but, preferably, it is carried out by the above-mentioned cell-free protein synthetic means or a translational/transcriptional non-coupling cell-free protein synthetic means of the present invention. The cell extract used for the cell-free protein synthesis may be that derived from *Escherichia coli,* reticulocyte of rabbit and wheat germ and, preferably, a wheat germ extract is used. When a wheat germ extract is used, it is preferred that the reaction is carried out under the static condition without stirring. In the Examples, co-expression was carried out using mRNA of five kinds of proteins and all of the proteins were confirmed to be simultaneously synthesized with a good efficiency.

The method of co-expression of plural proteins according to the present invention is useful since it can be used for the analysis of higher-order structure formation mechanism (co-folding) of plural proteins coupled with translation, for the preparation of samples for analysis of the relation between function and structure of the protein factor complex, for the preparation of samples in order to clarify the relation between gene mutation and cause of diseases, for the identification of target protein as medicament, for the preparation of samples for drug design, etc.

When the above-mentioned cell-free protein synthetic means, continuous transcriptional/translational non-coupling cell-free protein synthetic means and co-expression means for plural proteins according to the present invention are used, it is now possible to easily and efficiently synthesize the protein derived from living body, particularly from mammal, keeping the function which has been difficult to prepare by conventional method for the expression of protein using *Escherichia coli,* insect cells, etc.

Further, when the principles mentioned here are combined, it is possible to construct a fully automated cell-free protein synthetic apparatus which is a system starting from many kinds of aimed genes cloned into plasmids to prepare the proteins which is a translation product thereof at a time with a high efficiency.

Furthermore, when the means of the present invention is used, synthesis, etc. of many kinds of protein materials can be carried out efficiently and easily and, therefore, it is now possible to carry out the identification of gene function or analysis of function and structure of translation product, the analysis of the relation between gene polymorphism and gene function, the construction of screening method for gene function, the screening as well as the identification of target protein for reagents for clinical tests and therapeutic pharmaceuticals, the investigation of molecular design and efficacy of medicament using target protein.

### Examples

Hereinafter, the present invention will be illustrated by taking the synthesis of dhfr protein in a cell-free protein synthetic system utilizing a wheat germ extract as an example. However, the following examples should be considered as an aid for noting the specific examples of the present invention and the coverage of the present invention is never limited by the following examples.

### [Example 1]

### (Construction of 5'-terminal and 3'-terminal structures of mRNA having a high translation efficiency and structure of plasmid containing the said terminal structures)

dhfr Gene of *Escherichia coli* (Endo, Y., et al., (1992) *J. Biotech.*, 25, 221-230) was cloned by a conventional method into plasmid pPSP65 connected to SP6 promoter region to construct various plasmids where 5'-terminal and 3'-terminal non-translational sequence coding sites were modified (Table 1 and Table 3). "-dhfr-3'UTR" in upper line, left row, Table 1 shows the transcription product, "-" in left side shows 5'-UTR (36 bases) derived from dhfr gene and "-3'UTR" in right side shows 3'UTR comprising 539 bases. Various corresponding mRNA's having non-translational sequence were synthesized using those plasmid DNA of the original circular type or of a straight-chain type prepared by cleaving and opening with a restriction enzyme Hind III as a template, a translational template activity of each of them was measured and the result is shown in Table 1. mRNA was synthesized using DNA where the base sequence described in SEQ ID NO: 1, SEQ ID NO: 2 or SEQ ID NO: 3 of the Sequence Listing (corresponding to sequence-1, sequence-2 or sequence-3 in Table 2) was introduced into 5'-UTR instead of Ω sequence as a template, translational template activity of each of them was measured and the result is shown in Table 3.

An *in vitro* transcription of mRNA was carried out by a conventional method (Gurevich, V. V., (1996), *Methods in Enzymology,* 275, 383-397).

Conditions for this transcription reaction were as follows. Thus, the reaction solution {80 mM Hepes-KOH, pH 7.6, 16 mM magnesium acetate, 2 mM spermidine, 10 mM dithiothreitol (DTT), 2.5 mM each of nucleoside-5'-triphosphates [NTP; ATP, GTP, uridine-5'-triphosphate (UTP), cytidine-5'-triphosphate (CTP)], ribonuclease inhibitor (1,000 units/ml), plasmid DNA (50 µg/ml) and SP6 RNA polymerase (3,000 units/ml); incidentally, in the synthesis of RNA having a CAP structure at 5'-terminal, 5 mM of 7mGpppG were added to this solution}} was kept at 37°C for 2 hours to synthesize mRNA and, after extracting with phenol and precipitating with ethanol, the template DNA was digested, if necessary, by treating with a DNase. The precipitate with ethanol was dissolved in 10 mM Hepes-KOH (pH 7.6) and four kinds of NTP remaining in this fraction, 7mGpppG, degraded product of DNA and ion were removed by. means of a gel filtration column. mRNA was quantified by an ultraviolet absorption method (45 µg/l OD unit).

The translational template activity of the RNA synthesized as such was investigated using a cell-free protein synthetic system utilizing a wheat germ extract in a batch system. In that case, a cell-free protein synthesis utilizing a wheat germ extract was carried out according to the already-reported methods (Endo, Y. et al., (1992), *J. Biotech.,* 25, 221-230), (*Proc. Natl. Acad. Sci. USA*, (2000), 97, 559-564) and PCT/JP99/04088. However, selection of wheat germ was carried out by a method where good germs were selected according to their color by naked eye or under a microscope instead of a floatation method using cyclohexane or carbon tetrachloride mentioned therein. Fig. 1 shows the state of wheat germ used as a standard for the selection. Yellow germs as shown in Fig. 1(B) were principally collected while germs having small injury in white color and germs in brown and black colors as shown in Fig. 1(A) were discarded. Even by such a method, it is still possible to prepare a wheat germ extract having a sufficiently high translation activity and, in addition, there is no need for using toxic organic solvents such as cyclohexane and carbon tetrachloride.

To be specific, wheat germs used in a cell-free protein synthetic system were obtained by a modified method of Johnston, et al. (Johnston, F. B. et al., (1957), *Nature,* 179, 160-161). First, seeds (non-disinfected) of *chihoku* wheat produced in Hokkaido were added to a mill (Rotor Speed Mill Pulverisette type 14 manufactured by Fritsch) at the rate of 100 g per minute and was mildly crushed under the rotation of 8,000 rpm. They were crushed again at 6,000 rpm and sieved to give crude germ fraction (mesh size: 0.71 mm to 1.00 mm) and impurities such as seed coats contaminated in the germ fraction were removed by adsorbing with an electrostatically charged substance such as polyethylene plate.

Further, the germ particles were classified into small particles (0.71 mm to 0.85 mm), medium particles (0.85 mm to 1 mm) and light particles (0.85 mm to 1 mm and being light in weight) using a sieve and an electrostatically charged substance and, finally, classification by naked eye was conducted to collect yellow germs. Small particle fraction showed the highest protein synthetic activity. Light particles are assumed to be the product where destruction of the slightly injured germs resulted in the germs during crushing of the seeds proceeded during the operation of floating. Then, in order to remove the components of wheat germ endosperm from the sample, the wheat germs were placed in gauze, washed with cold distilled water (DDW) with cooling, suspended in a 0.5% solution of NP-40 which was a nonionic surface-active agent and repeatedly washed using an ultrasonic washing machine until the washing did not show turbidity. After conducting the ultrasonic washing once again in the presence of distilled water, the wheat germs were collected by filtration and aspiration, and purified by repeated washing with cold distilled water (DDW). In the wheat germs purified as such, endogenous protein synthesis-inhibiting factor, tritine, thionine, ribonuclease, etc. contaminating in the wheat germ were substantially removed showing no contamination with endosperm components.

Method for the preparation of an extract from wheat germ was in accordance with the conventional method (Erickson, A. H. et al., (1996) *Methods in Enzymol.,* 96, 38-50). The following operations were carried out at 4°C. Purified wheat germ frozen in liquid nitrogen was crushed in a mortar and an extracting solvent which was a partial modification of a method of Patterson, et al. (containing 80 mM of HEPES-KOH, pH 7.8, 200 mM of potassium acetate, 2 mM of magnesium acetate, 4 mM of calcium chloride, 8 mM of dithiothreitol, each 0.6 mM of 20 kinds of L-amino acids and each 1 µM of FUT, E-64 and PMSF which are protease inhibitors) was added in an amount of 1 ml to 1 g of the resulting powder followed by stirring carefully so as not to generate the foams. The supernatant obtained after centrifugation at 30,000 g for 15 minutes was collected as a germ extract and subjected to a gel filtration using a column of Sephadex G-25 (Coarse) which was previously equilibrated with a solution (40 mM of HEPES-KOH, pH 7.8, 100 mM of potassium acetate, 5 mM of magnesium acetate and 4 mM of dithiothreitol). Concentration of the sample was adjusted to 170∼250 A 260 nm (A260/A280 = 1.5).

Conditions for the synthesis of dhfr protein were as follows. The reaction solution contains 24% by volume of wheat germ extract (concentration: 200A260nm units/ml), 1,000 units/ml of ribonuclease inhibitor (RNAsin), 30 mM of Hepes-KOH, pH 7.6, 95 mM of potassium acetate, 2.65 mM of magnesium acetate, 2.85 mM of dithiothreitol, 0.5 mg/ml of creatine kinase, 1.2 mM of ATP, 0.25 mM of GTP, 16 mM of creatine phosphate, 0.380 mM of spermidine, 20 kinds of L-amino acids (0.3 mM each), 0.05% NP-40, 2 µCi (per ml of reaction volume) of [U-¹⁴C]leucine (166 mCi/mmol) and 0.2 µM of mRNA. The reaction was carried out under a static condition at 26°C and the protein synthetic activity was expressed in terms of the ratio (%) to the case where the radioactivity (2821 dpm/5 µl) of [U-¹⁴C]leucine incorporated in a dhfr protein using dhfr mRNA (being transcribed from a straight-chain type plasmid having 1167 base numbers; shown as * in Table 1) into which a CAP structure (7mGpppG) was introduced as a template at 5'-terminal was defined as 100%.

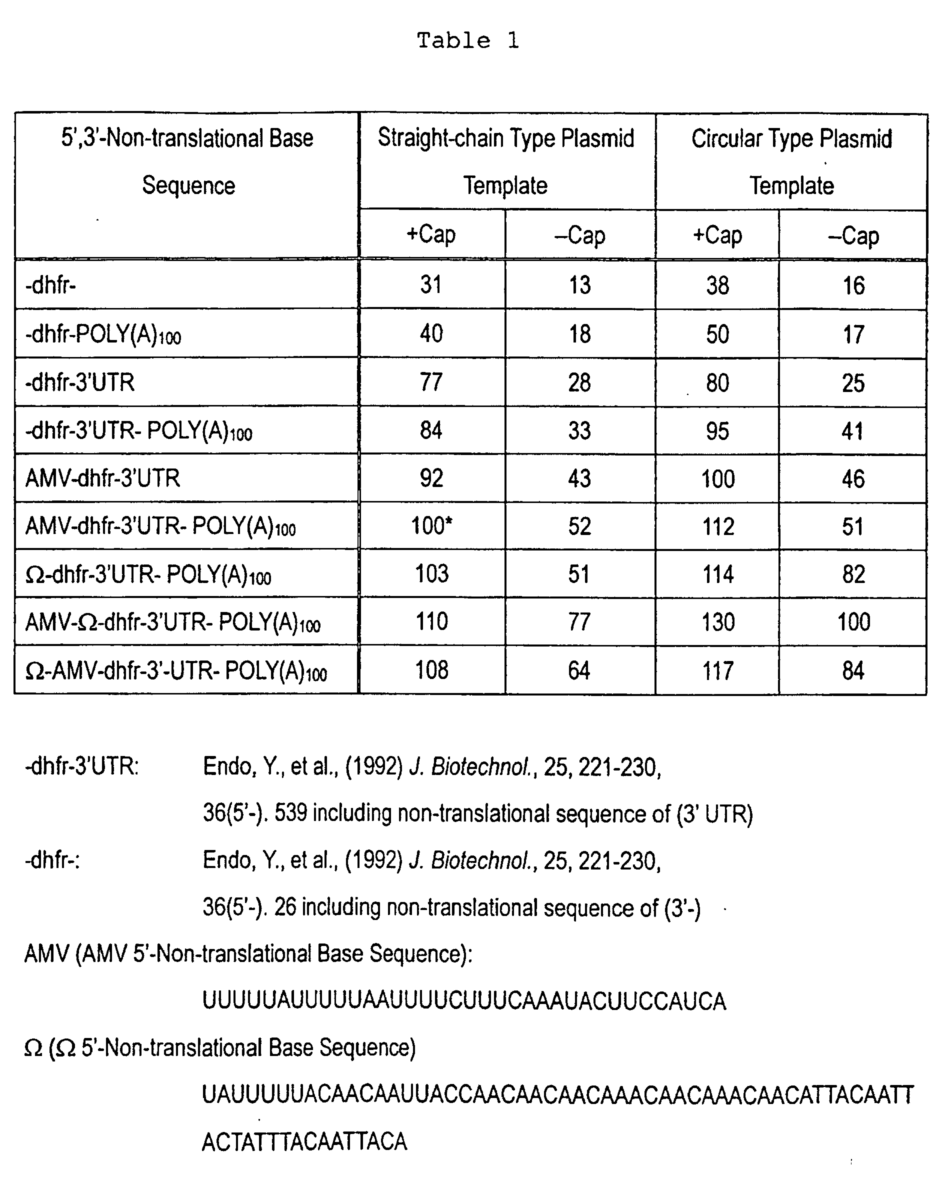

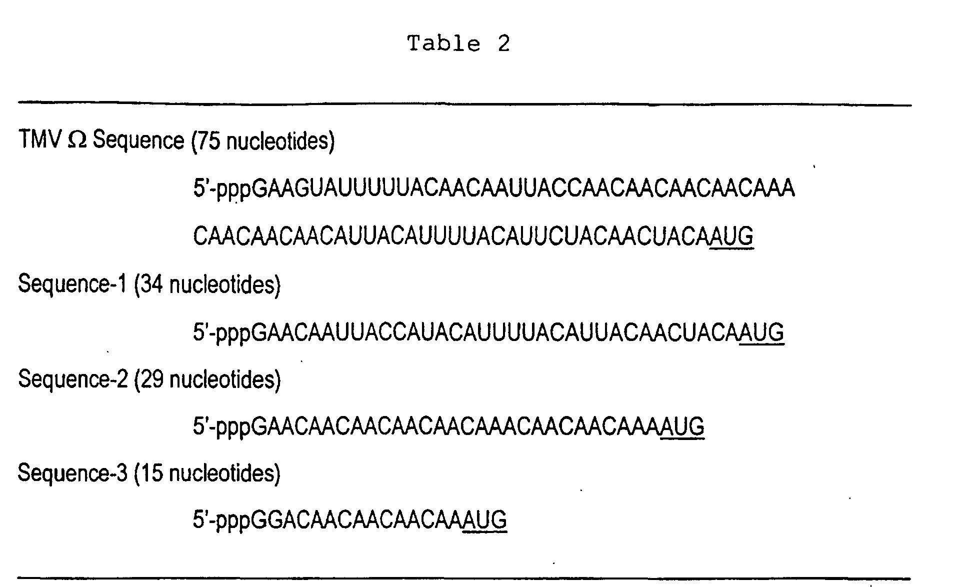

The underlined AUG shows a translation initiation codon.

**Table 3**

| 5'-UTR Structure | mRNA Concentration | Protein Synthetic Activity (dpm/5µl) (%) | | |
|---|---|---|---|---|
| | (µM) | 1 hour | 2 hours | 3 hours |
| TMVΩ Sequence | 1.80 | 1,569(100) | 2,288(100) | 2,615(100) |
| Sequence-1 | 1.80 | 1,113(71) | 1,967(86) | 2,301(88) |
| Sequence-2 | 1.80 | 1,036(66) | 1,785(78) | 2,170(83) |
| Sequence-3 | 1.80 | 596(38) | 1,235(54) | 1,412(54) |
| TMVΩ Sequence | 0.225 | 503(100) | 645(100) | 748(100) |
| Sequence-1 | 0.225 | 352(70) | 503(78) | 695(93) |
| Sequence-2 | 0.225 | 331(66) | 529(82) | 613(82) |
| Sequence-3 | 0.225 | 206(41) | 348(54) | 426(57) |

Base sequence of 3'-UTR of mRNA comprises the 539 bases described in Table 1 (Endo, Y. et al. (1992) *J. Biotech.,* 25, 221-230).

As will be apparent from the experimental results shown in Table 1, the AMV-Ω sequence in which, as a 5'-terminal non-translational sequence, 5'-terminal leader structure (AMV leader sequence) of alfalfa mosaic virus mRNA (AMV-mRNA) is linked in series to a 5'-terminal Ω sequence of tobacco mosaic virus mRNA (TMV-mRNA) showed the highest translational template activity. However, even in mRNA having an Ω-AMV sequence at the 5'-terminal, a significantly high translational template activity is maintained. It is noted on the other hand that, in any mRNA that where CAP is linked to 5'-terminal maintains nearly the same activity.

Table 3 shows the translational template activity in a batch reaction system of dhfr mRNA's having three kinds of 5'-UTR structures shown by the base sequences described in SEQ ID NO: 1, SEQ ID NO: 2 or SEQ ID NO: 3 of the Sequence Listing and any of them maintained a translational template activity which is as good as that having an Ω sequence.

A terminal polyadenylic acids addition in a 3'-terminal non-translational sequence is effective for potentiating the translational template activity but, with regard to the chain length added, there was no difference among 60, 80 and 100. In addition, the most noteworthy result is that the mRNA linked with RNA chain transcribed from a base sequence coded to the present plasmid at the downstream of the polyadenylic acids (mRNA transcribed using a circular type plasmid as a template) was found to maintain a very stable and high translation activity. Translation efficiency of the mRNA produced by the modified means according to the present invention (without CAP) was as good as the translation efficiency of the mRNA having a CAP structure at 5'-terminal.

As mentioned hereinabove, it has been clarified that, in order to obtain mRNA having a high translational template activity, it is effective that one of AMV leader sequence, TMV Ω sequence, a base sequence described in SEQ ID NO: 1, SEQ ID NO: 2 or SEQ ID NO: 3 of the Sequence Listing, etc. is linked or two or more thereof are linked together in series to the 5'-terminal non-translational sequence and further that a long-chain RNA containing polyadenylic acids is linked to the 3'-terminal. On the basis of those results, there was prepared pEU1 (Fig. 2) which is a plasmid exclusively for the template molecule synthesis for a cell-free protein synthesis equipped with a highly efficient translational template function having a broad applicability.

pEU1 is a plasmid in which plasmid pPAP65 is used as a substrate and a base sequence to which RNA polymerase will bind, such as SP6 promoter or T7 promoter is inserted, a base sequence acting as a transcriptional template of AMV-Ω sequence having an important function for translation initiation reaction of mRNA is inserted to its downstream and, further, plural restriction enzyme sites for introducing an aimed gene and 3'-terminal non-translational sequence containing poly(A) coding sequence are inserted to the downstream thereof. Moreover, there was prepared a plasmid into which a base sequence acting as a transcriptional template of a base sequence described in SEQ ID NO: 1, SEQ ID NO: 2 or SEQ ID NO: 3 of the Sequence Listing was inserted instead of an AMV-Ω sequence.

The straight-chain type plasmid of the present invention is that where the restriction enzyme site (Hind III site) introduced into the downstream of the poly (A) sequence of the present plasmid is cleaved by Hind III (Fig. 2). Incidentally, the number of 3'-terminal polyadenylic acid residues the mRNA used in the following Examples is 100 (poly(A)100) and the mRNA length transcribed using a straight-chain type plasmid as a template is 1.3 K base long (1,280 bases) . The mRNA transcribed using a circular type plasmid having a high translation efficiency as a template was a mixture of molecules having the chain lengths of 2.7 K, 7.4 K and 10.4 K base long [Fig. 3(B)]. Even in AMV-Ω-mRNA having a CAP structure, the translational template activity of mRNA where a long-chain RNA is linked to the 3'-terminal is high (Table 1) and that shows the long-chain RNA participates in the stabilization of mRNA.

### [Example 2]

### (Stabilizing means for mRNA by introduction of a long-chain 3'-terminal non-translational sequence)

As shown in Table 1, a template activity of mRNA is enhanced to an extent of several times when a long-chain RNA is introduced to the downstream of 3'-terminal non-translational sequence of mRNA. In order to check the cause, mRNA labeled with [³²P] was added to a cell-free protein synthetic system using a wheat germ extract and, using its radioactivity as an index, stability of mRNA in the translation reaction was checked. To be more specific, at the downstream of a 3'-terminal non-translational sequence of mRNA having an AMV-Ω sequence at 5'-terminal, the mRNA to which a long-chain RNA was linked having a different base length was designed and synthesized and stability in a cell-free protein synthetic reaction utilizing a wheat germ extract of those template molecules was investigated. With regard to a transcriptional template, a straight-chain type plasmid cleaved by Hind III or an untreated circular type plasmid was used and, in the presence of [α-³²P]UTP, synthesis and purification of mRNA with or without CAP structure were carried out by the same method as in Example 1. This labeled mRNA (0.2 µM) was used and a cell-free protein synthetic reaction was carried out by the method described in Example 1. With regard to the stability of mRNA, RNA was extracted by a phenol method after 0, 15, 30, 60 and 120 minute(s) of the reaction and purified by precipitating with ethanol, then separated by gel electrophoresis using 1% agarose and an autoradiography using BAS 2000 (Fuji Photo-Film) was carried out. A molecular size marker for mRNA and mRNA having a CAP structure synthesized by using straight-type plasmid as a transcriptional template obtained by cleaving with Hind III of each plasmid that is constructed by introducing SP6 or T7 promoter sequence to the promoter region were subjected to electrophoresis on the same gel.

From the experimental result shown in Fig. 3, it has been clarified that the long-chain RNA introduced into the 3'-terminal non-translational sequence is directly participated in the stabilization of mRNA molecule in a cell-free protein synthetic system. As will be apparent from Fig. 3, with regard to mRNA into which CAP structure is introduced at the 5'-terminal, both mRNA species transcribed from a straight-chain type plasmid [Fig. 3(A)] and from a circular type plasmid [Fig. 3(B)] are relatively high in their stability while the mRNA having no CAP structure at 5' -terminal [Fig. 3(A); the 5'-AMV-Ω at the right side; 1.3 K base long] is quite unstable and it is mostly degraded after one hour from the protein synthetic reaction. However, among the mRNA molecular species having no CAP structure transcribed from circular type plasmid, the mRNA having 2.7 K, 7.4 K and 10.4 K base long [Fig. 3(B); 5'-AMV-Ω at the right side] shows clearly higher stability than the mRNA having a 1.3 K base long and the stability of the molecules of 7.4 K and 10.4 K base long is as good as the stability of the mRNA having a CAP structure. Thus, one of the principles of the present invention that a long-chain RNA is introduced into a downstream of 3'-terminal non-translational sequence of mRNA has been proved to contribute in an increase of a template activity of mRNA caused by stability of mRNA.

In addition, the reason why the mRNA having the 5'AMV-Ω-dhfr-long-chain 3'-terminal non-translational sequence structure has a high translation efficiency is that, during the translation reaction, mRNA having a CAP structure produces degraded product by its degradation which strongly inhibits the translation initiation reaction while, in the mRNA of the present invention, no degraded product inhibiting the translation is produced. The non-translational sequence of RNA linked to the 3'-terminal of mRNA is not a particularly selected base sequence and, therefore, it has been clarified that the effect of promoting the translation efficiency of mRNA is not due to its base sequence itself but is due to a long chain of RNA linked to the 3'-terminal. The design principle of mRNA having such a high template activity can be applied for promoting the efficiency of the template activity of other mRNA species. As will be noted from the drawing shown as a molecular weight marker in Fig. 3(B) (drawing of the left side), this plasmid is able to synthesize the mRNA in the same manner either by the use of T7 promoter and T7 polymerase or by the use of polymerase and promoter of SP6 type. Fig. 2 shows a structure of plasmid for mRNA transcription having a high translational template activity for a cell-free protein synthesis and it is now possible to easily prepare mRNA having a high translational template activity without an operation of cleaving of DNA using a restriction enzyme when an aimed gene is introduced into this plasmid at any restriction enzyme site, the plasmid is amplified in the cells of *Escherichia coli* or the like and the plasmid prepared from the cells is used as a template.

### [Example 3]

### (Development of simple mass production and purifying technique of mRNA)

A microspin column (YM-10 manufactured by Amicon) where the bottom of the container was a dialysis filter was utilized and, as a transcriptional template, there was used a straight-chain type DNA where plasmid for dhfr mRNA synthesis in which AMV-Ω sequence was linked to a 5'-terminal while poly (A) 100 and 3'UTR derived from dhfr gene of *Escherichia coli* were linked to a 3'-terminal was cleaved with a restriction enzyme. Composition and reaction temperature of the RNA synthetic reaction solution are the same as those in Example 1.

A reaction solution (200 µl) was transferred to a reaction vessel washed with water and then the reaction was carried out by the conventional batch system [Fig. 4(A)] or, with dialyzing the reaction solution in the said reaction vessel to 2 ml of a transcription solution containing neither DNA nor RNA polymerase [Fig. 4(B)] or, with dialyzing in a similar manner using a reaction vessel which was coated with 10 mg/ml of bovine serum albumin after washing with water [Fig. 4(C)].

As shown in the lower drawing of Fig. 4, in the conventional batch type reaction [Fig. 4(A)], the reaction almost ceased within two hours and 0.23 mg of mRNA was synthesized per ml of the reaction volume. This yield was as same as that in the case of using a common test tube as a reaction vessel. On the other hand, in the dialyzing methods, the synthetic reaction continued for a long period and, after 8 hours, the yield increased up to 0.8 mg per ml of the reaction volume. However, as will be apparent from the electrophoretic drawing (upper drawing in Fig. 4), it was noted that degradation of mRNA took place together with elapse of time [Fig. 4(B)]. On the other hand, when a transcription was carried out with dialyzing using YM-10 which was coated with bovine serum albumin, the reaction proceeded at least for 16 hours and 1.8 mg of mRNA were able to be synthesized. This protein coating method is effective in increasing the transcription efficiency and in preventing the degradation of mRNA [Fig. 4(C)].

Although it is possible that purification of the synthesized mRNA is carried out by a conventional method of removal of protein using phenol and by precipitation of mRNA from an aqueous layer using ethyl alcohol or the like, it is also possible to carry out that simply by the following method.

Thus, the container (microspin column) itself is centrifuged after completion of the reaction, whereby low-molecular substances in the solution such as substrates (e.g, nucleoside triphosphate and CAP) and by-products (e.g., pyrophosphoric acid, ion and buffer) are removed by passing through the filter pores and the high-molecular substances such as the synthesized mRNA remained on the filter. When water is added to the container and the operation is repeated for several times, the synthesized mRNA is collected on the filter whereby it is now possible to remove low-molecular protein synthesis inhibitors such as pyrophosphoric acid from the fraction containing the synthesized mRNA.

Although it is also possible to synthesize protein by adding a filter on which mRNA is collected as it is to a cell-free protein synthetic system, mRNA may be further recovered as an aqueous solution from the filter to use. The present method is similarly applicable to the mRNA synthesis having a CAP structure as well.

### [Example 4]

### (Synthetic method for mRNA having a high translational template activity using a circular type plasmid as a template)

In order to further confirm the high translation efficiency of dhfr mRNA where a combination of AMV and Ω sequence was linked in series as a 5'-terminal non-translational sequence and non-translational sequence and polyadenylic acids were moreover linked to the 3'-terminal as shown in Example 1, there was synthesized mRNA using a straight-chain type plasmid or circular type plasmid for the synthesis of dhfr mRNA where AMV-Ω sequence was linked to the 5'-terminal and poly(A)100 was linked to the 3'-terminal by an RNA synthetic method with dialysis described in Example 3. There was furthermore synthesized mRNA to which poly(A) was linked to the end of 5'-CAP-dhfr-3'-terminal by the dialysis method in the same manner. After purification by a centrifugal method, an appropriate volume of water was added and mRNA was recovered from a filter as an aqueous solution.

Translational template activity of those mRNA's was investigated by the same method as in Examples 1 and 3 using a cell-free protein synthetic system utilizing a wheat germ extract in a batch system (where mRNA was synthesized by transcribing from a template plasmid, purified and added to the reaction solution as a translational template to carry out the reaction) (Endo, Y. et al., (1992) *J. Biotech.,* 25, 221-230), (*Proc. Natl. Acad. Sci. USA* (2000), 97, 559-564) (Fig. 5). The coordinate shows the radioactivity (dpm/5µl) incorporated into the protein which was synthesized until 3 hours from the start of a protein synthetic reaction when linear progress of the reaction is seen while the abscissa shows the mRNA concentration in the reaction solution. As a result, it was found that mRNA transcribed from a circular type plasmid, wherein a long-chain RNA is linked to the 3'-terminal [a mixture of mRNA's having chain lengths of 2.7 K, 7.4 K and 10.4 K bases shown in Fig. 3(B)] ( 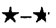 ), maintained higher template activity at a 0.6 µM concentration of mRNA in about twice as much than that ( 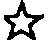 - 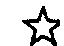 ) from a straight-chain plasmid. The translation activity at that time was 87% of mRNA (●-●) having a CAP structure showing the maximum activity. Thus, the mRNA where a combined structure of AMV and Ω sequence was linked in series to the 5'-terminal non-translational sequence and, further, a long-chain RNA containing polyadenylic acids was linked to the 3'-terminal showed a high translational template activity. It was also proved that such mRNA can be easily prepared by a method of synthesis and purification using dialysis where a circular type plasmid was used as a transcriptional template.

Further, as will be apparent from Fig. 5, mRNA having a CAP structure showed a sudden inhibition of protein synthesis when it was more than the optimum concentration of 0.5 µM while mRNA having no CAP structure synthesized using a circular type plasmid as a template did not show a strong inhibiting action even at 1.8 µM. From those facts, it is understood that those translation inhibition phenomena noted in mRNA having a CAP structure is caused by an antagonistic inhibitor effect due to a competitive binding of the degraded product of mRNA containing CAP molecule to the factor for protein synthesis initiation reaction. Thus, in 5'-AMV-Ω mRNA having no CAP structure transcribed from circular type plasmid where a long-chain RNA is linked to the 3'-terminal, there is no such an antagonistic inhibition and, therefore, the range of the optimum template concentration in a cell-free protein synthetic system is far broad and setting of the condition for the protein synthetic reaction is easy. Thus, it seems that the mRNA shown here achieves to retain a high efficiency as a translational template by means of having both 5'-terminal non-translational sequence having a high translation initiation activity and a long-chain structure of 3'-terminal participating in stability. Further, cleaving with a restriction enzyme is not carried out in such a transcription method using a circular type plasmid and, therefore, it is unrelated to the presence of the base sequence being recognized by the restriction enzyme in the translation base sequence (ORF) in the aimed gene and any species of mRNA is able to be synthesized as a full-length molecule having a high translation efficiency.

### [Example 5]

### (Continuous cell-free protein synthesis using dhfr mRNA where AMV-Ω sequence is linked to 5'-terminal and poly(A)100 is linked to 3'-terminal transcribed from circular type plasmid)

Next, properties of the mRNA synthesized by the continuous transcription were investigated in a continuous cell-free protein synthetic system. A continuous cell-free protein synthesis using a dialysis membrane was carried out using mRNA molecular species having a 5' AMV-Ω structure where mRNA was synthesized using straight-chain type and circular type plasmids as templates by a dialytic method described in Example 3 and, after the protein was separated by means of an SDS-gel electrophoresis, staining with Coomassie Blue was carried out. Each mRNA was added at the initiation of the reaction only. The concentration was 2 µM each. The result is shown in Fig. 6. Fig 6(A) and Fig. 6(B) are the results when mRNA synthesized using circular type plasmid and straight-chain type plasmid, respectively, as templates were used as translational templates. An arrow on gel shows dhfr protein which is a translation product while a star thereon shows creatine kinase in the reaction solution.

As will be apparent from Fig. 6, it is noted from the stained intensity of the dhfr protein which is a translation product that, in a cell-free protein synthetic system using mRNA transcribed from a circular type plasmid, the translation reaction continues for longer than 40 hours [Fig. 6(A)] while, in the case of mRNA synthesized from a straight-chain type plasmid, the reaction stops within 10 hours from the initiation of the reaction [Fig. 6(B)]. When amount of the synthesized dhfr protein was quantified by densitometry using the intensity of stained band analyzed by SDS-PAGE in the same manner by adding the known amount of dhfr protein to the unreacted cell-free protein synthetic solution as a standard, it was found that the mRNA synthesized using the circular type plasmid showed nearly the same high yield as in the case where the template used was AMV-Ω-dhfr-3'-terminal poly(A)100-linked dhfr mRNA having a CAP structure at 5'-terminal. On the other hand, the translational template activity of mRNA transcribed from a straight-chain plasmid was one-half or even less of the efficiency of the circular type one. Thus, a long-chain dhfr mRNA (synthesized using a circular type plasmid as a template) transcribed from a circular type plasmid where AMV-Ω sequence was linked to the 5'-terminal and poly (A) 100 was linked to the 3' -terminal maintained a stable and high template activity even in a continuous cell-free protein synthetic system whereby its efficacy has now been confirmed.

The fact that the mRNA transcribed from a circular type plasmid showed better efficiency as a translational template than the mRNA synthesized from a straight-chain type plasmid does not only show such a thing but also shows other better usefulness. Thus, when a straight-chain type plasmid is used as a transcriptional template as in the conventional case, plasmid is cleaved at the restriction enzyme site which is at the 3' downstream of the inserted gene prior to the transcription reaction and, in that case, it is necessary to confirm that the restriction enzyme recognizing structure is absent in the translated base sequence (ORF) of the aimed gene and, if that is present, it is necessary to construct a plasmid having another restriction enzyme site at the 3' downstream.

However, even when a restriction enzyme recognizing 6 bases is used, the calculation teaches that the same base sequence as the restriction enzyme site appears in a frequency of every 4096 bases and, therefore, a straight-chain type plasmid is unable to be utilized for the cloning of many kinds of genes for transcription. For example, in about 100,000 types of gene in human being, the calculation teaches that about 20,000∼30,000 base sequences which are identical with restriction enzyme sites are present in ORF when average nucleotide numbers in the gene are assumed to be 1200 and, therefore, in view of the nature of the restriction enzyme which has been known at present, it is principally impossible to construct a straight-chain plasmid for the transcription of mRNA.

In the circular type plasmid shown here, it is not necessary to cleave the plasmid using a restriction enzyme prior to the transcription reaction if cloning is carried out to a plasmid for the synthesis of novel cell-free protein synthesis (a cassette plasmid by which an addition of AMV-Ω sequence and poly(A)100 to the 5'-terminal and the 3'-terminal of mRNA, respectively is possible) and, therefore, transcription of mRNA from all aimed genes using one kind of cassette plasmid is possible. In other words, it is now possible to synthesize a protein encoded by such genes in a cell-free protein synthetic system.

### [Example 6]

### (Continuous cell-free protein synthetic method utilizing the mRNA collected on the filter as it is)

mRNA was synthesized by a simple method for mass production and purification of mRNA as described in Example 3, then extraction with phenol was not carried out but the reaction vessel was centrifuged whereupon the purified mRNA collected on the filter was taken out from a spin column together with the filter, the filter was poured into an inner area of dialysis membrane filled with a protein synthetic reaction solution and a continuous cell-free protein synthesis was carried out. The filter collecting the mRNA was added only at the initiation of the reaction. Concentration of mRNA calculated from the amount of mRNA on the filter and from the reaction volume was about 2 µM.

As shown in Fig. 6(C), it has been found that a continuous cell-free protein synthetic system to which a filter collecting the dhfr mRNA where AMV-Ω sequence is linked to the 5' -terminal and poly(A)100 is linked to the 3'-terminal transcribed from the circular type plasmid achieves a stable protein synthetic ability for a long period and maintains the highest translational template activity. It has been also confirmed that mRNA can be preserved for a long period in a state of being collected on the filter.

### [Example 7]

### (A united system for a continuous RNA synthesis and a continuous cell-free protein synthesis - a continuous transcriptional/translational non-coupling cell-free protein synthetic method utilizing a dialysis membrane -)

A circular type plasmid for the synthesis of a long-chain dhfr mRNA where a base sequence described in SEQ ID NO: 1 of the Sequence Listing was linked to the 5'-terminal and poly(A)100 was linked to the 3'-terminal was added to a transcription reaction solution (refer to Example 1) and was subjected to a preliminary incubation at 30°C for 5 minutes. Its object is to make the succeeding transcription reaction by RNA polymerase efficient. After the preliminary incubation, the transcription reaction solution and the protein synthetic solution (refer to Example 1) were mixed and transferred into an inner area of dialysis membrane. In this example, the transcription reaction solution and the translation reaction solution were mixed in a ratio of 2:1 by volume and, therefore, concentrations of magnesium ion and four kinds of ribonucleoside-5'-triphosphates at the initiation of the reaction were 11.7 mM of UTP, 1.67 mM of CTP, 2.07 mM of ATP and 1.76 mM of GTP and so on. With regard to the external solution for dialysis, a solution for a continuous cell-free protein synthetic reaction (refer to Examples 1 and 11) (Endo, Y. et al., (2000) *Proc. Natl. Acad. Sci. USA*, 97, 559-564) (PCT/JP99/04088) was used, mRNA was synthesized in large quantities by incubating at 30°C for 2 hours, temperature of the reaction system was lowered to 23°C and protein synthesis using a dialysis membrane continuous system was carried out as shown in Fig. 7(B). Analysis was carried out by means of an SDS-gel electrophoretic method as same as in Example 5. In the drawing, an arrow shows dhfr protein which is a translation product while a star shows creatine kinase in the reaction solution.

Under the present reaction condition, an mRNA synthesis proceeds in the initial stage of the reaction when the composition of the reaction solution is suitable for the transcription reaction and, when the solution composition of the solution in the inner area of dialysis membrane changes to a composition of the translation reaction as a result of a progress of dialysis via a dialysis membrane, a protein synthetic reaction is initiated and proceeds. The result is shown in Fig. 8(A) and 4 mg of dhfr protein per ml of the reaction solution was able to be synthesized under a static reaction condition. Incidentally, the reason why the said protein synthetic method is achieved is a cell-free protein synthetic solution utilizing a wheat germ extract used is quite stable. The present principle and means are different from the already-invented and commercially-available transcriptional/translational reaction coupling system (U. S. Patents No. 5,665,563, No. 5,492,817 and No. 5,324,637) in terms of the principle. As will be apparent from Fig. 8(A), the present system achieves a stable protein synthetic ability for a long period whereby it is understood that the present system is an easy and efficient protein synthetic means. Since the present system is simple in terms of the principle and the operation, it is applicable for the construction of a fully automated cell-free protein synthetic apparatus by which an automatic synthetic of protein from many kinds of genes is possible.

### [Example 8]

### (A united system of a continuous RNA synthesis and a continuous cell-free protein synthesis - a continuous transcriptional/translational non-coupling cell-free protein synthetic method using pure mRNA collected on a filter as a template -)

mRNA was synthesized by a method described in Example 3 and purified by centrifugation without an extracting treatment with phenol, a cell-free protein synthetic solution was added to a spin column in a state where mRNA was collected on a filter [Endo, Y. et al., (1992) *J. Biotech.,* 25, 221-230], [*Proc. Natl. Acad. Sci. USA* (2000), 97, 559-564] and a continuous cell-free protein synthesis was carried out according to a method of PCT/JP99/04088 and Examples 1 and 11 [Fig. 7(A)]. Analysis was carried out by means of an SDS-gel electrophoretic method as same as in Example 5. In the drawing, an arrow shows a dhfr protein which is a translation product while a star shows creatine kinase in the reaction solution.

As shown in Fig. 8(B), it has been found that a continuous cell-free protein synthetic system using a filter column collecting a long-chain dhfr mRNA (mRNA synthesized using a circular type plasmid as a template) transcribed from a circular type plasmid, where a base sequence described in SEQ ID NO: 2 of the Sequence Listing is linked to the 5'-terminal and poly(A)100 is linked to the 3'-terminal, achieves a stable protein synthesizing ability for a long period and maintains a high translational template activity. The synthetic yield of dhfr by this method per ml of the reaction solution was 3 mg. This method is a continuous cell-free protein synthetic method in which transcription and translation are not coupled and is applicable in the same manner to a protein synthesis using mRNA having a CAP structure at 5' -terminal as a template as well. Further, when a multiple type spin column where many columns having a filter are accumulated is prepared, synthesis of protein from many kinds of genes at a time is possible and, since its principle and operation are simple, it is now possible to construct a fully automated cell-free protein synthetic apparatus in which an automatic liquid-sending and centrifugal devices are integrated.

### [Example 9]

### (Production of osteopontin)

In order to prepare a fused protein (GST-OPN) of osteopontin (OPN) with GST, plasmids shown in Fig. 9(A) and Fig. 9(B) were designed in a similar manner to Examples 1 and 2. GST was fused with OPN so that it was easily purified by affinity chromatography using a glutathione column after the synthesis of GST-OPN. Osteopontin is a kind of adhesive protein contained in matrix of bone, called as a secretory phosphate protein 1 and has a molecular weight of about 69,000 and cDNA of rat was cloned in 1985. Masato Nose (Faculty of Medicine, Ehime University), et al. have found that gene locus encoding this substance is a candidate gene for glomerular nephritis. In order to investigate the relation between gene polymorphism of OPN (*Mol. Immunol.* (1995) 32, 447-448) and onset of glomerular nephritis, OPN gene derived from MRL/lpr mouse (having defective mutation gene of Fas, lpr) which is a model mouse for collagen disease and causes glomerular nephritis and OPN gene derived from C3H/lpr mouse which is a normal control are prepared from a cDNA library prepared from splenocytes of each mouse and then pET-GST-C3H and pEU-GST-MRL as shown in Fig. 9(A) and Fig. 9(B) were designed and constructed in a similar manner to Example 2 by a conventional method. Any of them has a structure having a transcription promoter (Pro) for SP6 polymerase, a base sequence which is to be a transcriptional template for AMV-Ω sequence as 5'-UTR, GST-OPN as ORF and a polyA(100) coding sequence at 3'-terminal. In the drawings, PreScission™ is a protease cleaving site manufactured by Pharmacia. Molecular weight of C3H type OPN (OPN/C3H) of matured type is 31,118 daltons while that of MRL type OPN (OPN/MRL) of a matured type is 30,987 daltons. With regard to the molecular weight of designed fused proteins, the former is 57,746 daltons (GST-OPN/C3H) and the latter is 57,553 daltons (GST-OPN/MRL).

### (Synthesis of GST-OPN by a batch type cell-free protein synthetic system)

mRNA was transcribed and synthesized using the above-constructed circular type plasmid as a template, the said mRNA was purified and, using it as a translational template, synthesis of a fused type OPN with GST was carried out under a static condition using a wheat germ extract similar to that of Example 1 in a batch system in a similar manner to Example 3. The said mRNA was added in an amount of 4 µg per 25 µl of the reaction solution for the protein synthesis. As same as in Example 1, synthesized amount of protein is expressed as an incorporated amount of radioactivity to a trichloroacetic acid fraction in Fig. 10 where a coordinate shows the incorporated amount per 5 µl of the reaction solution in terms of dpm values. Both fused proteins derived from MRL/lpr and from C3H/lpr were synthesized almost linearly until 2 hours. Incidentally, dhfr is a control experiment for confirming that the reaction system normally functions.

### (Autoradiography of a synthesized fused OPN)

In Fig. 11, molecular weight of a fused protein up to 2 hours synthesized hereinabove is confirmed and it has been confirmed that the resulting protein is a designed protein. The bigger mobility of GST-OPN/C3H than GST-OPN/MRL reflects the steric structures in the presence of SDS caused by the difference (polymorphism) of the amino acid sequences of both.

### (Preparation of OPN/C3H and OPN/MRL)

Fig. 12 is a result where the mass synthesis by a dialytic method (refer to Example 3) in which transcription from mRNA and translational synthesis of protein were continuously carried out was investigated by an SDS-polyacrylamide gel electrophoresis. From a stained pattern with Coomassie Brilliant Blue (CBB), synthesis of GST-OPN/C3H (lane T) and that of GST-OPN/MRL (lane T) were able to be confirmed and the former was 1.2 mg while the latter was 1.4 mg per ml reaction volume. Each OPN was purified using a kit manufactured by Pharmacia whereupon it was confirmed from the analytical result that fused proteins (each lane E) and OPN/C3H as well as OPN/MRL (each lane D) showed the molecular weights as designed. Although not shown in the drawing, it was also possible that HIS-OPN/C3H and HIS-OPN/MRL synthesized as fused proteins using histidine oligomer (HIS-tag) instead of GST as a tag for purification were purified to an extent of high purity using a nickel affinity chromatography using nickel as a ligand.

### (Bioassay using synthesized OPN)

Splenocytes were collected from MRL/+ mouse and C3H/+ mouse of eight weeks age and were suspended in an RPMI 1640 medium. To this was added each of the above-synthesized OPN to give 10 ng/ml and a short incubation was carried out for 24 hours or 48 hours. mRNA was extracted from the cells after incubation and an RT-PCR was carried out using a primer which was specific to each of IgG3, IL-2 and IL-10 whereby the expression thereof was quantitatively analyzed. The result was that the synthesized OPN of an MRL type (OPN/MRL) induced the production of IgG2a and IgG3 in splenetic cells and the effect was higher than the OPN of the C3H type (OPN/C3H). That suggested the difference in structural protein and function thereof due to polymorphism of OPN structural gene has a possibility of participating in the onset of glomerular nephritis in MRL/lpr mouse via a promotion of antibody production. It has been also confirmed that, according to the cell-free protein synthetic system of the present invention, protein which maintains a function and is able to function *in vivo* can be synthesized by gene derived from mammals. As a result, it has been found that the cell-free protein synthetic system utilizing a wheat germ is able to be used as a means for testing the relation between gene polymorphism and diseases in a level of gene-protein expression.

### [Example 10]

### (Co-expression of plural proteins)

A cell-free protein synthetic system utilizing a wheat germ was utilized and, in the co-presence of many translational templates, many kinds of the corresponding proteins were synthesized at the same time. The proteins subjected to a co-expression were the following five types: dihydrofolate reductase (dhfr, 18 kDa), glutathione-S-transferase (GST, 28 kDa), green fluorescent protein (GFP, 27 kDa), GST-osteopontin fused protein (G-OPN, 67 kDa) and luciferase (LUC, 61 kDa).

First, in accordance with Example 2, there were constructed each of the plasmids for mRNA synthesis of each protein containing SP6 promoter, a base sequence which is to be a transcriptional template for AMV-Ω sequence as 5'-UTR, ORF coding for each protein and poly(A)100 coding sequence as 3'-UTR. After each mRNA which was transcriptionally synthesized and purified using the plasmid was added separately to a dialyzing cell-free protein synthetic system (refer to Example 11) utilizing a wheat germ extract, a protein synthetic reaction was carried out for 120 hours under a static condition and each of the synthetic results is shown in Fig. 13(A). The result is expressed by the stained result of the protein separated by an SDS-PAGE using CBB. As shown in Fig. 13(A), each protein was synthesized in a high yield when synthesized solely. However, when five kinds of mRNA's were added at the same time and synthesis was carried out by a dialyzing cell-free protein synthetic system utilizing the same wheat germ extract, only a little amount of GFP were synthesized while no other protein was synthesized [lane MIX of Fig. 13(A)]. The reason therefor was presumed to be that not only the structures of 5'-and 3'-UTR but also base sequences of ORF encoding the amino acid sequence and numbers thereof have an influence on the translation initiation efficiency. Thus, each of the five kinds of mRNA's was added to each of the tubes for protein translation reaction, subjected to a preliminary incubation (pre-incubation) for 3 hours, so that each mRNA was separately subjected to initiation of translation efficiently and, after that, they were mixed and a dialysis reaction was carried out similarly. As shown in Fig. 13(B), it has been clarified that, in such a method, all of the five proteins can be efficiently synthesized. With regard to G-OPN, although it shows a molecular weight of 67 kDa, its mobility on an SDS-polyacrylamide gel is low and is not separated from a band of an endogenous protein of wheat germ.

### [Example 11]

### (Continuous cell-free protein synthesis by a dialysis method utilizing a wheat germ extract)

Method for the synthesis of a continuous cell-free protein synthesis utilizing a wheat germ extract was carried out in a similar manner to the known report (Endo, Y. et al. (1992) *J. Biotech.,* 25, 221-230), the reaction solution of the pure wheat germ was placed in a dispo-dialyzer (Spectra/Por CE®, MWCO: 25 k, volume: 0.5 ml) and the reaction was carried out under a static condition at 20°C in a dialysis system using 10-fold by volume (to the reaction solution) of an external solution for dialysis (20 mM of HEPES-KOH, pH 7.6, 95 mM of potassium acetate, 2.65 mM of magnesium acetate, 4 mM of dithiothreitol, 1.2 mM of ATP, 0.25 mM of GTP, 16 mM of creatine phosphate, 0.38 mM of spermidine, 20 kinds of L-amino acids [0.3 mM each], 0.005% of NaN₃, 0.05% of NP-40 and each 1 mM of E-64 and PMSF). According to that method, various proteins having molecular weights of 5,000∼130,000 can be efficiently synthesized and the synthetic efficiency per ml of the reaction volume was 0.3∼1.8 mg. When the synthetic reaction was carried out under a stirring condition using a stirrer at 100∼140 rpm, the protein synthesis efficiency was lower than the case when carried out under the static condition. Fig. 14(A) and Fig. 14 (B) show the electrophoretic graphs when GFP was synthesized as a synthesized protein. Fig. 14(A) was under the static condition without stirring while Fig. 14 (B) was the case where stirring was done using a stirrer at 120 rpm. As will be apparent from the difference in the GFP synthetic amount as shown by the concentration of the bands in the electrophoretic graph, no increase in the synthetic amount took place under the stirring condition even when the reaction was continued for 48 hours. Thus, it has been now been proved that, in a cell-free protein synthetic system utilizing a wheat germ extract, it is essential to carry out the reaction under a static condition.

### Industrial Applicability

In the present invention, there were disclosed construction of 5'-terminal and 3'-terminal non-translational sequences of translational template mRNA for an efficient cell-free protein synthesis, construction of plasmid for mRNA transcription having broad applicability, method for synthesis and of purification of mRNA having a high translational template activity and examples of application thereof for a practical cell-free protein synthesis such as a continuous transcriptional/translational non-coupling cell-protein synthetic means comprising a continuous mRNA synthesis and a continuous cell-free protein synthesis utilizing a wheat germ extract and also a method for co-expression of plural proteins utilizing the cell-free protein synthetic means. The present invention achieved a less expensive, easy and efficient RNA synthesis and, as a result, it achieved the offer of a less expensive, easy and efficient cell-free protein synthetic means. In accordance with the present invention, synthesis of protein maintaining the function *in vivo* which has been difficult to obtain by the conventional protein expressing method using *Escherichia coli*, insect cells, etc. is now able to be carried out easily and efficiently. Consequently, the present invention can be utilized for identification of genetic function or, in other words, analysis of function and structure of translation products, analysis of the relation between gene polymorphism and genetic function, construction of a method for the screening of genetic function, screening and identification of target protein for reagent for clinical tests and therapeutic medicaments, investigation of molecular design and effectiveness of medicaments using a target protein, etc. whereby it highly contributes in a pharmaceutical field and is extremely useful.

## Claims

1. A modifying means for translational template molecule which is a modifying means for translational template molecule being able to be used as a means for cell-free protein synthesis comprising that at least one base sequence selected from the following, i.e.
1) a base sequence having an alfalfa mosaic virus (AMV) leader sequence,
2) a base sequence having an Ω sequence of tobacco mosaic virus (TMV),
3) a base sequence having 70% or more homology to an alfalfa mosaic virus (AMV) leader sequence and being able to give mRNA which has about 70% or more translation initiation activity as compared with the translation initiation activity of mRNA where a CAP structure is present,
4) a base sequence having 70% or more homology to an Ω sequence of tobacco mosaic virus (TMV) and being able to give mRNA which has about 70% or more translation initiation activity as compared with the translation initiation activity of mRNA where a CAP structure is present,
5) a base sequence described in SEQ ID NO: 1, SEQ ID NO: 2 or SEQ ID NO: 3 of the Sequence Listing and
6) a base sequence having 70% or more homology to the sequence selected from the base sequences described in SEQ ID NO: 1 to NO: 3 of the Sequence Listing and being able to give mRNA which has about 70% or more translation initiation activity as compared with the translation initiation activity of mRNA where a CAP structure is present
is introduced into a structure of 5'-terminal of the said translational template molecule and then at least a non-translational sequence is introduced into a structure of 3'-terminal.

2. The modifying means for translational template molecule according to claim 1, wherein there is combined with an introduction of a base sequence having a known translation initiation activity into a 5'-terminal.

3. A plasmid utilized for the manufacture of a translational template molecule used for a cell-free protein synthetic means wherein
① a base sequence carrying a promoter function,
② a base sequence at the downstream thereof which is to be a transcriptional template for at least
an alfalfa mosaic virus (AMV) leader sequence,
an Ω sequence of tobacco mosaic virus (TMV),
a base sequence having 70% or more homology to an alfalfa mosaic virus (AMV) leader sequence or an Ω sequence of tobacco mosaic virus (TMV) and being able to give mRNA having about 70% or more translation initiation activity as compared with the translation initiation activity of mRNA where a CAP structure is present,
a base sequence described in SEQ ID NO: 1, SEQ ID NO: 2 or SEQ ID NO: 3 of the Sequence Listing or
a base sequence having 70% or more homology to the base sequence described in SEQ ID NO: 1, SEQ ID NO: 2 or SEQ ID NO: 3 of the Sequence Listing and being able to give mRNA which has about 70% or more translation initiation activity as compared with the translation initiation activity of mRNA where a CAP structure is present,
③ plural restriction enzyme cleavage sites at the region corresponding to the downstream thereof and
④ at least 3' -terminal non-translational sequence at the region corresponding to the downstream of the termination codon of ORF (open reading frame)
are aligned.

4. The plasmid according to claim 3, wherein the size of the plasmid body is 3 Kb or more,

5. The plasmid according to claim 3 or 4, wherein the plasmid is a circular type,

6. The plasmid according to claim 5, wherein a base sequence carrying a transcription terminator function is aligned at the upstream of a base sequence carrying a promoter function.

7. The plasmid according to claim 3 or 4, wherein the plasmid is a straight-chain type .

8. A method for the synthesis of RNA utilizing the means according to claim 1 or 2.

9. The method for the synthesis of RNA according to claim 8, wherein it is **characterized in that**, in a reaction system for the synthesis of RNA, a substance necessary for the synthesis of the said RNA is continuously supplied and/or a substance inhibiting the synthesis of the said RNA and the by-product of the reaction are continuously removed.

10. A method for the synthesis of RNA used for a cell-free protein synthetic means using any of the plasmids according to claims 3 to 7.

11. The method for the synthesis of RNA according to claim 10, wherein it is **characterized in that**, in a reaction system for the synthesis of RNA, a substance necessary for the synthesis of the said RNA is continuously supplied and/or a substance inhibiting the synthesis of the said RNA is continuously removed.

12. A cell-free protein synthetic means utilizing the mRNA obtained by any of the synthetic methods for RNA according to claims 8 to 11.

13. A cell-free protein synthetic means utilizing any of the plasmids according to claims 3 to 7.

14. A cell-free protein synthetic means where the cell-free protein synthetic means according to claim 12 or 13 is a cell-free protein synthetic means using an extract of wheat germ and, in the said wheat germ extract, contamination of endosperm components is substantially removed.

15. A continuous transcriptional/translational non-coupling cell-free protein synthetic means which is the cell-free protein synthetic means according to claim 12 or 13, wherein it is **characterized in that** the mRNA synthesis (transcription) and the protein synthesis (translation) are not coupled but are continuously carried out in a system.

16. A continuous transcriptional/translational non-coupling cell-free protein synthetic means where the protein synthesis (translation) can be carried out in the same reaction vessel which is a cell-free protein synthetic means utilizing the plasmid according to claim 5 or 6 comprising that the mRNA synthesis (transcription) is carried out in a reaction vessel having a molecular sieve carrier, the said synthesized mRNA is purified and collected by a carrier which can carry out a molecular sieving and, under a dialysis condition, temperature of the reaction solution containing mRNA in the reaction vessel is changed to a temperature appropriate for the protein synthesis so that the protein synthetic reaction of the mRNA is carried out.

17. A continuous transcriptional/translational non-coupling cell-free protein synthetic means which is a cell-free protein synthetic means utilizing the plasmid according to claim 5 or 6, wherein, a reaction vessel having a dialyzing means is used and, after the mRNA synthesis (transcription) is carried out in an inner area of dialysis membrane, the temperature of the reaction solution is changed to a temperature which is appropriate for the protein synthesis (translation) and, the solution composition in the inner area of dialysis membrane is converted to a solution composition for the protein synthesis by dialysis whereby the mRNA synthesis (transcription) and the protein synthesis (translation) can be carried out in the same reaction vessel.

18. A continuous transcriptional/translational non-coupling cell-free protein synthetic means comprising the following steps:
(a) mRNA is synthesized in a reaction vessel having a carrier which is able to carry out a molecular sieving,
(b) the said synthesized mRNA is purified by the carrier which is able to carry out a molecular sieving and then collected and
(c) the protein synthesis is carried out in a reaction vessel containing the said synthesized and collected mRNA under a dialysis condition.

19. A continuous transcriptional/translational non-coupling cell-free protein synthetic means comprising the following steps:
(1) there is used a reaction vessel having a dialysis membrane where a mixed solution of the transcription reaction solution and the translation reaction solution is added to the inner area of the dialysis membrane,
(2) a solution for the protein synthesis reaction is filled in as an external solution for dialysis to manufacture the mRNA in an inner area of dialysis membrane,
(3) then the temperature of the reaction solution is changed to a temperature which is suitable for the protein synthesis reaction (translation reaction) and
(4) synthesis of protein is carried out under a dialysis condition.

20. A continuous transcriptional/translational non-coupling cell-free protein synthetic means where, in the continuous transcriptional/translational non-coupling cell-free protein synthetic means according to any of claims 15 to 19, an element which is selected at least from mRNA which is to be a translational template for protein synthesis reaction, enzyme of an energy regeneration system, substrate and energy source is subjected to a treatment selected from addition, preservation, exchange and discharge.

21. A cell-free protein synthetic means, **characterized in that**, a protein coating is applied at least on the surface participating in the dialyzing means or the molecular sieving means among the surfaces which are contacting to the reaction solution in the reaction vessel wherein mRNA synthesis is carried out.

22. The continuous transcriptional/translational non-coupling cell-free protein synthetic means according to any of claims 16, 18, 20 and 21, wherein, as a molecular sieving means or a carrier which is able to carry out a molecular sieving, there is carried out using a filter having a pore size whereby separation of the synthesized mRNA from low-molecular substances such as substrate and by-products is possible.

23. A continuous transcriptional/translational non-coupling cell-free protein synthetic means wherein the cell-free protein synthetic system used in the continuous transcriptional/translational non-coupling cell-free protein synthetic means according to any of claims 15 to 22 is a system utilizing a wheat germ extract and, in the said wheat germ extract, contamination of endosperm components is substantially removed.

24. A method for the co-expression of plural proteins using a cell-free protein synthetic means, **characterized in that**, two or more mRNA's which are translational templates in a cell-free protein synthetic means are used and each mRNA is separately pre-incubated in a cell-free protein synthetic reaction solution followed by mixing to conduct a cell-free protein synthesis.

25. The method for the co-expression of plural proteins according to claim 24, wherein the mRNA used is an mRNA which is synthesized by any of the synthetic methods for RNA according to claims 8 to 11.

26. The method for the co-expression of plural proteins according to claim 24 or 25, wherein the cell-free protein synthetic means is a cell-free protein synthetic means using a wheat germ extract and contamination of the endosperm components in the said wheat germ extract is substantially removed.

27. A wheat germ material for the cell-free protein synthesis comprising only yellow germ where germ having small injured area having white color and germ having brown or black color are removed.

28. A wheat germ extract which is manufactured from the wheat germ material according to claim 27.

29. The cell-free protein synthetic means according to claim 14, wherein the wheat germ extract where contamination of endosperm components is substantially removed is the wheat germ extract according to claim 28.

30. The continuous transcriptional/translational non-coupling cell-free protein synthetic means according to claim 23, wherein the wheat germ extract where contamination of endosperm components is substantially removed is the wheat germ extract according to claim 28.

31. The method for the co-expression of plural proteins according to claim 26, wherein the wheat germ extract where contamination of endosperm components is substantially removed is the wheat germ extract according to claim 28.

32. A cell-free protein synthetic means, **characterized in that**, in a cell-free protein synthetic system using a wheat germ extract, the cell-free protein synthetic reaction is carried out under such a condition that a reaction solution containing the wheat germ extract is not stirred under a static condition.

33. The cell-free protein synthetic means according to claim 14 or 29, wherein the cell-free protein synthetic reaction is carried out under such a condition that a reaction solution containing the wheat germ extract is not stirred under a static condition.

34. The continuous transcriptional/translational non-coupling cell-free protein synthetic means according to claim 23 or 30, wherein the cell-free protein synthetic reaction is carried out under such a condition that a reaction solution containing the wheat germ extract is not stirred under a static condition.

35. The method for the co-expression of plural proteins according to claim 26 or 31, wherein the cell-free protein synthetic reaction is carried out under such a condition that a reaction solution containing the wheat germ extract is not stirred under a static condition.

36. A method for the test of the relation between gene polymorphism and gene function, **characterized in that**, one means/method selected from the cell-free protein synthetic means according to claims 12∼14, 29 and 33, the continuous transcriptional/translational non-coupling cell-free protein synthetic means according to claims 15∼23, 30 and 34 and the method for the co-expression of plural proteins according to claims 24∼26, 31 and 35 is utilized.

37. A method for the screening of the gene function, **characterized in that**, one means/method selected from the cell-free protein synthetic means according to claims 12∼14, 29 and 33, the continuous transcriptional/translational non-coupling cell-free protein synthetic means according to claims 15∼23, 30 and 34 and the method for the co-expression of plural proteins according to claims 24∼26, 31 and 35 is utilized.

38. A method for the synthesis of protein using one means/method selected from the cell-free protein synthetic means according to claims 14, 29 and 33, the continuous transcriptional/translational non-coupling cell-free protein synthetic means according to claims 23, 30 and 34 and the method for the co-expression of plural proteins according to claims 26, 31 and 35, **characterized in that**, protein is synthesized from gene derived from mammal utilizing a wheat germ extract.
